# EUROPEAN PATENT SPECIFICATION

(11) **EP 1 753 763 B1**
(45) Date of publication and mention of the grant of the patent: **13.08.2008**
(21) Application number: 05742759.3
(22) Date of filing: 17.05.2005
(51) Int. Cl.: C07D 471/04

(54) **INDAZOLE AND INDOLONE DERIVATIVES AND THEIR USE AS PHARMACEUTICALS**
INDAZOL- UND INDOLONDERIVATIVE UND DEREN VERWENDUNG ALS PHARMAZEUTIKA
DERIVES INDAZOLE ET INDOLONE ET LEUR UTILISATION PHARMACEUTIQUE

(30) Priority: 26.05.2004 GB 0411810; 09.07.2004 GB 0415455
(43) Date of publication of application: 21.02.2007
(73) Proprietor: Pfizer Limited, Sandwich, Kent CT13 9NJ (GB); Pfizer, Inc., New York, NY 10017 (US)
(72) Inventor: ALLERTON, Charlotte Moira Norfor, Sandwich, Kent CT13 9NJ (GB); HEPWORTH, David, Sandwich, Kent CT13 9NJ (GB); MILLER, Duncan Charles, Sandwich, Kent CT13 9NJ (GB)
(74) Representative: Motion, Keith Robert
(86) International application number: PCT/IB2005/001513
(87) International publication number: WO 2005/116027

(56) References cited:
- WO-A-93/23035
- WO-A-98/35942
- WO-A-03/051370
- WO-A-20/04052372
- BETTINETTI L ET AL: "Interactive SAR studies: rational discovery of super-potent and highly selective dopamine D3 receptor antagonists and partial agonists" 6 September 2002 (2002-09-06), JOURNAL OF MEDICINAL CHEMISTRY, AMERICAN CHEMICAL SOCIETY. WASHINGTON, US, PAGE(S) 4594-4597 , XP002256409 ISSN: 0022-2623 the whole document

## Description

The present invention relates to a class of dopamine agonists, more particularly a class of agonists that are selective for D3 over D2. These compounds are useful for the treatment and/or prevention of sexual dysfunction, for example female sexual dysfunction (FSD), in particular female sexual arousal disorder (FSAD), hypoactive sexual desire disorder (HSDD; lack of interest in sex), female orgasmic disorder (FOD; inability to achieve orgasm); and male sexual dysfunction, in particular male erectile dysfunction (MED). Male sexual dysfunction as referred to herein is meant to include ejaculatory disorders such as premature ejaculation, anorgasmia (inability to achieve orgasm) or desire disorders such as hypoactive sexual desire disorder (HSDD; lack of interest in sex). These compounds are also useful in treating neuropsychiatric disorders and neurodegenerative disorders.

The present invention provides for compounds of formula (1), 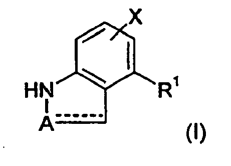 wherein:
A is selected from N or C=O
X is selected from H, methyl, ethyl, OH, OCH₃, OCH₂CH₃, halo, SCH₃, CN or CF₃
----- (the dashed bond in formula (I)) represents a single bond when A is N, and is absent when A is C=O
R¹ is selected from: 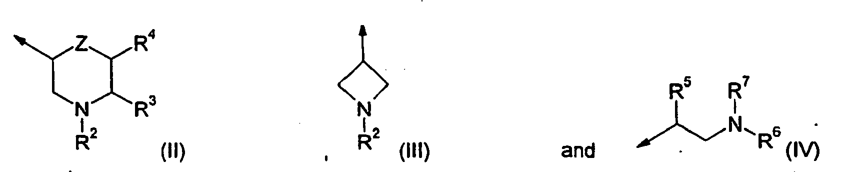
wherein:
Z represents O or CH₂;
R² represents H or (C₁-C₆)alkyl; wherein said (C₁-C₆)alkyl may be optionally substituted by (C₁-C₆)alkyl, OR⁸, phenyl, or heteroaryl;
R³ represents H or (C₁-C₆)alkyl; wherein said (C₁-C₆)alkyl may be optionally substituted by OR⁸;
R⁴ represents H or (C₁-C₆)alkyl;
R⁵ represents H, methyl, ethyl, methoxy, or ethoxy;
R⁶ represents (C₁-C₆)alkyl;
R⁷ represents H or (C₁-C₆)alkyl; wherein said (C₁-C₆)alkyl may be optionally substituted with 1 or 2 substituents each independently selected from OR⁸, phenyl or substituted phenyl;
R⁸ represents H, (C₁-C₆)alkyl, phenyl, or (CH₂)phenyl;
wherein heteroaryl means a 5 to 7 membered aromatic ring, containing from 1 to 4 heteroatoms, said heteroatoms each independently selected from O, S and N; said heteroaryl may be optionally substituted with 1 or more substituents selected from (C₁-C₆)alkyl, halo and OR⁸, each substituent may be the same or different; and
wherein substituted phenyl means phenyl substituted with 1 or more substituents selected from (C₁-C₆)alkyl, halo and OR⁸, each substituent may be the same or different;
and pharmaceutically acceptable salts, solvates, polymorphs and thereof, with the provisos that:
Z is O when A is C=O; and
when A is C=O, X is H and R¹ is (IV), then R⁵ cannot be H.

Unless otherwise indicated, (C₁-C₆)alkyl may be straight chain or branched.

Suitable heteroaryl groups include pyridinyl, pyrimidinyl, pyridazinyl and pyrazinyl.

Unless otherwise indicated, the term halo means fluoro, chloro, bromo or iodo.

Unless otherwise indicated, the term substituted means substituted by one or more defined groups. In the case where groups may be selected from a number of alternatives groups, the selected groups may be the same or different.

The pharmaceutically acceptable salts of the compounds of the formula (I) include the acid addition salts thereof.

A pharmaceutically acceptable salt of a compound of the formula (I) may be readily prepared by mixing together solutions of a compound of the formula (I) and the desired acid. The salt may precipitate from solution and be collected by filtration or may be recovered by evaporation of the solvent.

Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, 2002).

Pharmaceutically acceptable salts of compounds of formula I may be prepared by one or more of three methods:
(i) by reacting the compound of formula I with the desired acid;
(ii) by removing an acid- or base-labile protecting group from a suitable precursor of the compound of formula I or by ring-opening a suitable cyclic precursor, for example, a lactone or lactam, using the desired acid; or
(iii) by converting one salt of the compound of formula I to another by reaction with an appropriate acid or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in a continuum of solid states ranging from fully amorphous to fully crystalline. The term 'amorphous' refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterised by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterised by a phase change, typically first order ('melting point').

The compounds of the invention may also exist in unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water.
The pharmaceutically acceptable solvates of the compounds of the formula (I) include the hydrates thereof.

A currently accepted classification system for organic hydrates is one that defines isolated site, channel, or metal-ion coordinated hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

Hereinafter all references to compounds of formula I include references to the salts and solvates thereof.

The compounds of the invention include compounds of formula I as hereinbefore defined, including all polymorphs and crystal habits thereof, prodrugs and isomers thereof (including optical, geometric and tautomeric isomers) as hereinafter defined and isotopically-labeled compounds of formula I.

Included within the scope of the present invention are all stereoisomers, geometric isomers and tautomeric forms of the compounds of formula I, including compounds exhibiting more than one type of isomerism, and mixtures of one or more thereof. Also included are acid addition or base salts wherein the counterion is optically active, for example, *d*-lactate or *l*-lysine, or racemic, for example, *dl*-tartrate or *dl-*arginine.

A compound of the formula (I) contains one or more asymmetric carbon atoms and therefore exists in two or more stereoisomeric forms. Furthermore, the skilled person will understand that moiety. (II) encompasses all stereoisomeric and distereoisomeric forms, in particular:

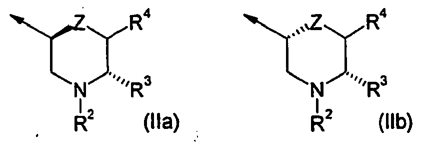

Separation of diastereoisomers may be achieved by conventional techniques, e.g. by fractional crystallisation, chromatography or H.P.L.C. of a stereoisomeric mixture of a compound of the formula (I) or a suitable salt or derivative thereof. An individual enantiomer of a compound of the formula (I) may also be prepared from a corresponding optically pure intermediate or by resolution, such as by H.P.L.C. of the corresponding racemate using a suitable chiral support or by fractional crystallisation of the diastereoisomeric salts formed by reaction of the corresponding racemate with a suitable optically active acid or base, as appropriate.

The present invention includes all pharmaceutically acceptable isotopically-labelled compounds of formula I wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³l and ¹²⁵l, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of formula I, for example, those incorporating a radioactive isotope, are useful in drug and/or substrate tissue distribution studies. The radioactive isotopes tritium, *i.e.* ³H, and carbon-14, *i.e.* ¹⁴C , are particularly useful for this purpose in view of their ease of incorporation and ready means of detection.

Substitution with heavier isotopes such as deuterium, *i.e.* ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of formula I can generally be prepared by conventional techniques known to those skilled in the art or by processes analogous to those described in the accompanying Examples and Preparations using an appropriate isotopically-labeled reagent in place of the non-labeled reagent previously employed.

Pharmaceutically acceptable solvates in accordance with the invention include those wherein the solvent of crystallization may be isotopically substituted, *e.g.* D₂O, d₆-acetone, d₆-DMSO.

### The following embodiments of the invention are particularly favoured:

Preferably X is selected from H, methyl, OH, OCH₃, F, Cl, CN or CF₃
More preferably X is selected from H, OH, F, or CN
Most preferably X is H

Preferably heteroaryl is selected from pyridinyl, pyrimidinyl, pyridazinyl and pyrazinyl; each optionally substituted with 1 or more substituents selected from (C₁-C₆)alkyl, halo and OR⁸, each substituent may be the same or different.

More preferably heteroaryl is selected from pyridinyl and pyrimidinyl; each optionally substituted with 1 or more substituents selected from (C₁-C₆)alkyl, halo and OR⁸, each substituent may be the same or different.

Most preferably heteroaryl is selected from pyridinyl and pyrimidinyl; each optionally substituted with 1 or 2 substituents selected from methyl, fluoro, chloro and methoxy, each substituent may be the same or different.

When R¹ is represented by moiety (II):
Moieties (IIa) and (IIb) are preferred.
Moiety (IIa) is particularly preferred.

Preferably Z represents O.
Preferably R² represents (C₁-C₆)alkyl.
More preferably R² represents methyl, ethyl or propyl.
Most preferably R² represents n-propyl.

Preferably R³ represents H or (C₁-C₆)alkyl.
More preferably R³ represents H, methyl, ethyl or propyl.
Most preferably R³ represents H, methyl or ethyl.

Preferably R⁴ represents H or (C₁-C₄)alkyl.
More preferably R⁴ represents H, methyl or ethyl.
Most preferably R⁴ represents H.

When R¹ is represented by moiety (III):

Preferably A is N.

Preferably R² represents (C₁-C₄)alkyl, optionally substituted by phenyl or heteroaryl.
More preferably R² represents ethyl, propyl or butyl, each optionally substituted by phenyl.
Most preferably R² represents ethyl or propyl, each optionally substituted by phenyl.

When R¹ is represented by moiety (IV):

Preferably A is N.

Preferably R⁵ is selected from H, methyl and methoxy.
More preferably R⁵ is selected from H and methoxy.
Most preferably R⁵ is H.

Preferably R⁶ is selected from (C₁-C₄)alkyl.
More preferably R⁶ is selected from methyl, ethyl and n-propyl.
Most preferably R⁶ is n-propyl.

Preferably R⁷ is selected from H and (C₁-C₃)alkyl; wherein said (C₁-C₃)alkyl may be optionally substituted with 1 or 2 OR⁸ or phenyl groups.
More preferably R⁷ is selected from H and methyl.
Most preferably R⁷ is H.

Preferably R¹ is selected from moieties (II) and (IV)
More preferably R¹ is selected from moieties (IIa), (IIb), and (IV)
Most preferably R¹ is selected from moieties (IIa) and (IV).

Preferably R⁸ represents H, (C₁-C₄)alkyl, phenyl, or (CH₂)phenyl
More preferably R⁸ represents H, methyl, or (CH₂)phenyl
Most preferably R⁸ represents H or (CH₂)phenyl

Particularly preferred are compounds (and salts thereof) of the present invention exemplified herein; more preferred are:
4-[(2*R*,5*S*)-5-methyl-4-propylmorpholin-2-yl]-1H-indazole (Example 1)
4-[1-(2-ethyl)azetidin-3-yl]-1*H*-indazole (Example 7)
4-[1-(3-phenylpropyl)azetidin-3-yl]-1*H*-indazole (Example 10)
4-(1-propylazetidin-3-yl)-1*H*-indazole (Example 13)
4-[(2*R*)-4-propylmorpholin-2-yl]-1,3-dihydro-2*H*-indol-2-one (Example 14).
4-[(2*R*,5*S*)-5-methyl-4-propyl-morpholin-2-yl]-1,3-dihydro-indol-2-one (Example 17)
*N*-[2-(1*H*-indazol-4-yl)ethyl]-*N*-propylamine (Example 18)
4-[(2*R*,5*S*)-5-methyl-4-ethylmorpholin-2-yl]-1H-indazole and
4-[(2*S*,5*S*)-5-methyl-4-ethylmorpholin-2-yl]-1*H*-indazole (Examples 19 and 20)

Compounds of the invention may be prepared, in known manner, in a variety of ways. The routes below illustrate methods of synthesising compounds of formula (I).

Throughout the transformations of the following schemes, any suitable nitrogen protecting groups may be used (as described in "Protecting Groups in Organic Synthesis" 3rd Edition T. W. Greene and P.G. Wuts, Wiley-Interscience, 1999). A common nitrogen protecting group (PG) suitable for use herein is tert-butoxy carbonyl, which is readily removed by treatment with an acid such as trifluoroacetic acid or hydrogen chloride in an organic solvent such as dichloromethane. A particularly suitable nitrogen protecting group for use with the indazoles herein described is triphenylmethyl (trityl), which is readily removed by treatment with an acid such as trifluoroacetic acid, sulfuric acid or hydrogen chloride in an organic solvent such as dichloromethane. A particularly suitable nitrogen protecting group for use with the indoles herein described is triisopropylsilyl (TIPS), which is readily removed by treatment with an acid such as sulfuric acid in an organic solvent such as dichloromethane.

The skilled man will appreciate that, in addition to protecting nitrogen groups, as discussed hereinbefore, at various times during the synthesis of the compounds of formula I, it may be necessary to protect further groups, such as for example, hydroxy groups with a suitable protecting group, then remove the protecting group. Methods for deprotection of any particular group will depend on the protecting group. For examples of protection/deprotection methodology see "Protective groups in Organic synthesis", TW Greene and PGM Wutz. For example, where a hydroxy group is protected as a methyl ether, deprotection conditions comprise refluxing in 48% aqueous HBr for 1-24 hours, or by stirring with borane tribromide in dichloromethane for 1-24 hours. Alternatively where a hydroxy group is protected as a benzyl ether, deprotection conditions comprise hydrogenation with a palladium catalyst under a hydrogen atmosphere.

Compounds of formula (I) wherein R² and X are as defined above and, A and R¹ are as described herein, may be prepared according to reaction scheme 1.

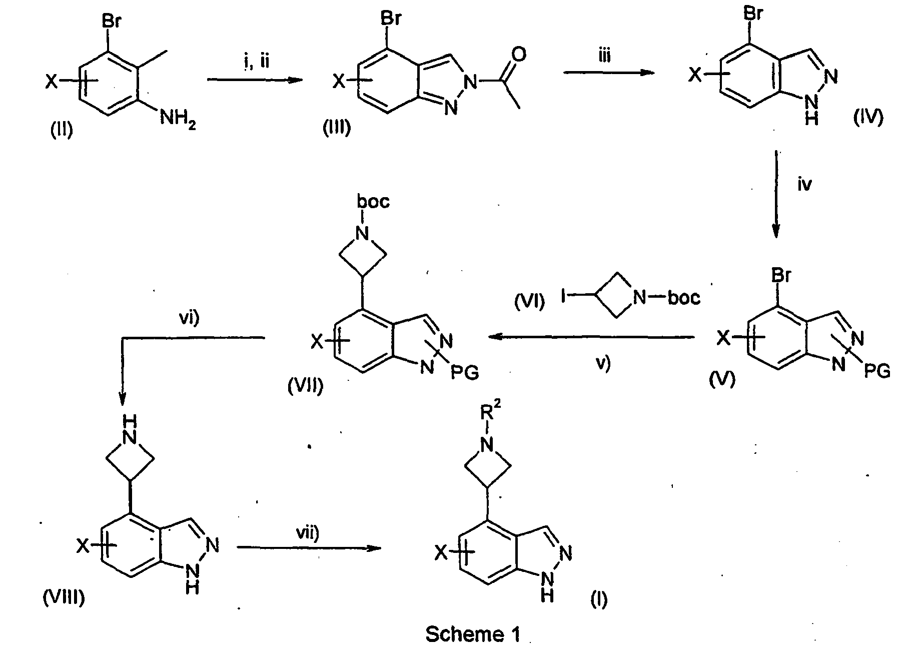

Compounds of formula (III) may be prepared by reacting compounds of formula (II) with i) Ac₂O in the presence of KOAc, followed by treatment with ii) isoamylnitrite. Typical conditions comprise 1 equivalent of the aniline (II) with 3 equivalents of Ac₂0 and 1-1.5 equivalents of KOAc using toluene as solvent at 60°C for 30 minutes, followed by treatment with isoamylnitrite (1.5 equivalents). Compounds of formula (II) are commercially available.

Compounds of formula (IV) may be prepared by reacting compounds of formula (III) with iii) a suitable acid. Typical conditions comprise 1 equivalent of the acylindazole (III) as a solution in 5M aqueous HCl being treated with excess concentrated hydrochloric acid at a temperature of 50°C for 15 minutes followed by stirring at 60°C for 15 minutes.

Compounds of formula (V), wherein, for example, PG is trityl, may be prepared by reacting compounds of the formula (IV) with iv) chlorotriphenylmethane or bromotriphenylmethane in the presence of a base. Typical conditions comprise 1 equivalent of the indazole (IV) with 1 equivalent of chlorotriphenylmethane and 1-3 equivalents of triethylamine using dichloromethane as solvent at room temperature for 18 hours.

Compounds of formula (VII) may be prepared by reacting compounds of the formula (VI) with v) Zn/Cu couple with sonication, followed by addition of compounds of the formula (V) and a suitable palladium catalyst and ligand, and heating to 70°C for 18 hours. Typical conditions comprise 1 equivalent of the azetidine (VI) with 40 wt% Zn/Cu couple in DMF with sonication at room temperature for 4 hours, followed by addition of 1.05 equivalents of the indazole (V), 0.05 equivalents of tris(dibenzylideneacetone)dipalladium(0) and 0.1 equivalents of tri-o-furylphosphine and heating to 70 °C for 18 hours. Compounds of the formula (VI) may be prepared as described in *Synlett,* 4, 1998, 379.

Compounds of formula (VIII) may be prepared by reacting compounds of the formula (VII) with vi) a suitable acid, such as HCl or TFA in a suitable solvent such as dichloromethane or diethyl ether at room temperature or above, preferably in the presence of a cation scavenger such as Et₃SiH. Typical conditions comprise 1 equivalent of the indazoleazetidine (VII) with excess trifluoroacetic acid and 1.5 equivalents of Et₃SiH in CH₂Cl₂ at room temperature for 90 minutes.

Compounds of formula (I) may be prepared by reacting compounds of formula (VIII) with vii) 1-5 equivalents of the required aldehyde in a suitable solvent at room temperature in the presence of 1-5 equivalents of a suitable reducing agent such as sodium triacetoxyborohydride or sodium cyanoborohydride in a suitable solvent such as dichloromethane or tetrahydrofuran with the optional addition of acetic acid. Typical conditions comprise 1 equivalent of the indazoleazetidine (VIII) with 3.1 equivalents of the aldehyde and 3.1 equivalents of sodium triacetoxyborohydride in dichloromethane at room temperature for 18 hours.

Compounds of formula (I) wherein X, R², R³ and R⁴ are as defined above and A, R¹ and Z are as described herein, may be prepared according to reaction scheme 2.

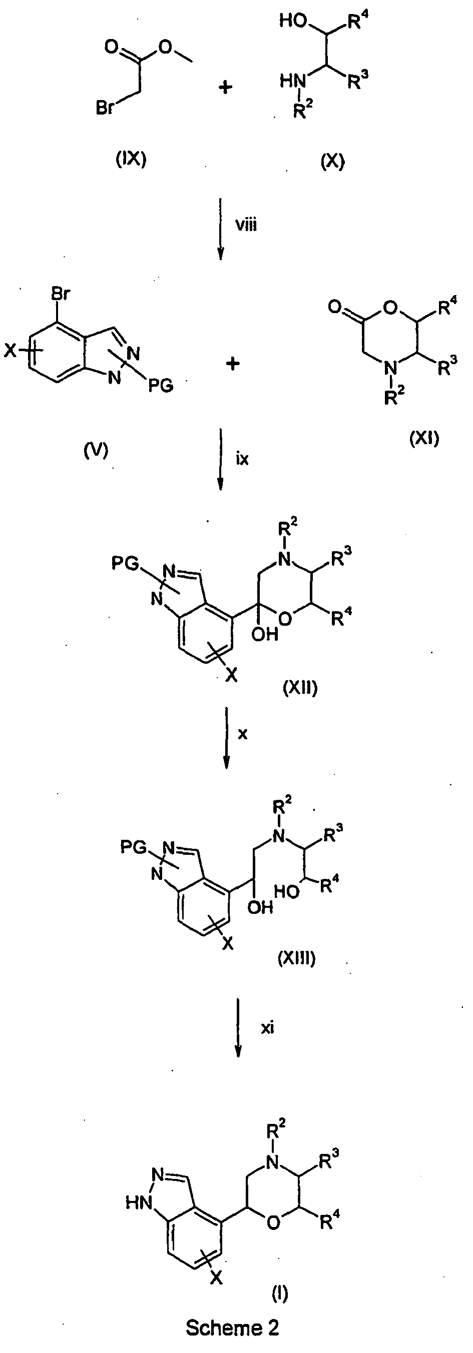

Compounds of formula (XI) may be prepared by reacting compounds of formula (IX) with viii) amino alcohols of formula (X) in the presence of a base such as triethylamine or 4-methylmorpholine. Typical conditions comprise 1 equivalent of the aminoalcohol (X) with 1-3 equivalents of triethylamine and 1 equivalent of a compound of formula (IX) using toluene as solvent at room temperature or above. Compounds of formula (IX) are commercially available.

Compounds of formula (XII) may be prepared by reacting a compound of formula (XI) with ix) an organometallic reagent formed from the bromide (or similar iodide) of formula (V). Suitable organometallic reagents include Grignard (organomagnesium) or organolithium reagents, which may be prepared from the bromide by halogen metal exchange. Typical conditions comprise addition of *t*-butyllithium (1.7M in pentane) to the bromide (V) in an anhydrous ethereal solvent such as tetrahydrofuran at a temperature below -70°C (to perform the halogen metal exchange reaction), followed by addition of the morpholinone (XI).

Morpholinol (XII) may be reduced to diol (XIII) by x) reaction with a hydride reducing agent, such as sodium borohydride in an alcohol solvent such as methanol. Typical conditions comprise addition of 4 equivalents of the reducing agent to the morpholinol (XII) in aqueous ethanol at room temperature.

Compounds of formula (I) may be prepared from the diol (XIII) by xi), deprotection and cyclisation in the presence of a suitable acid. Typical conditions comprise 1.0 equivalents of compound (XIII) and excess concentrated H₂SO₄ in dichloromethane at room temperature or above.

Compounds of formula (I) wherein X, R², R³ and R⁴ are as defined above and A, Z and R¹ are as described herein, may be prepared according to reaction scheme 3.

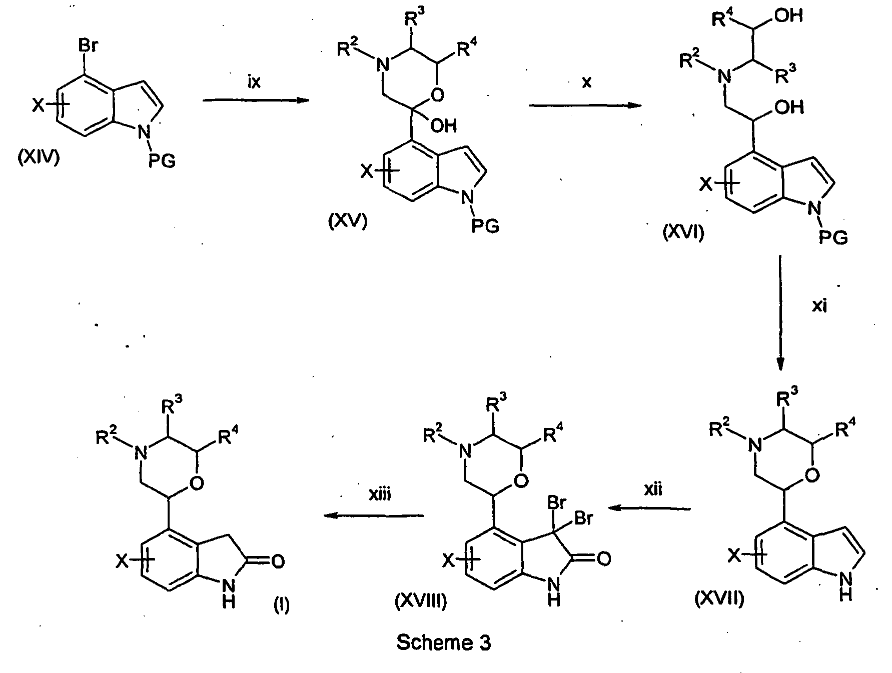

Compounds of formula (XIV), wherein, for example, PG is TIPS, may be prepared from 4-bromoindole by reaction with a triisopropylsilyl halide in the presence of a suitable base. Typical conditions comprise addition of the base to 4-bromoindole in THF as solvent, at reduced temperature, followed by addition of triisopropylsilyl chloride and then heating to reflux. 4-bromoindole is commercially available.

Compounds of formula (XV) may be prepared by reacting a compound of formula (XI) with ix) an organometallic reagent formed from the bromide of formula (XIV). Suitable organometallic reagents include Grignard (organomagnesium) or organolithium reagents, which may be prepared from the bromide by halogen metal exchange. Typical conditions comprise addition of *t*-butyllithium (1.7M in pentane) to the bromide (XIV) in an anhydrous ethereal solvent such as tetrahydrofuran at a temperature below -70°C (to perform the halogen metal exchange reaction), followed by addition of the morpholinone (XI).

Compounds of formula (XVI) may be prepared by reduction of morpholinol (XV) by x) reaction with a hydride reducing agent, such as sodium borohydride in an alcohol solvent such as methanol. Typical conditions comprise addition of 4 equivalents of the reducing agent to the morpholinol (XV) in aqueous ethanol at room temperature.

Indoles of formula (XVII) may be prepared from the diol (XVI) by xi), deprotection and cyclisation in the presence of a suitable acid. Typical conditions comprise 1.0 equivalents of compound (XVI) and excess concentrated H₂SO₄ in dichloromethane at room temperature or above.

Compounds of formula (XVIII) may be prepared from indoles of formula (XVII) by xii) addition of pyridinium tribromide in a suitable solvent. Typical conditions comprise adding 3 equivalents of pyridinium tribromide to the indole of formula (XVII) in *t*-butanol at room temperature.

Compounds of formula (I) may be prepared from compounds of formula (XVIII) by xiii) addition of zinc dust in a suitable solvent. Typical conditions comprise adding 10 equivalents of zinc dust to the compound of formula (XVIII) in glacial acetic acid at room temperature.

Compounds of formula (I) wherein X, R², R³ and R⁴ are as defined above and A, Z and R¹ are as described herein, may be prepared according to reaction scheme 4.

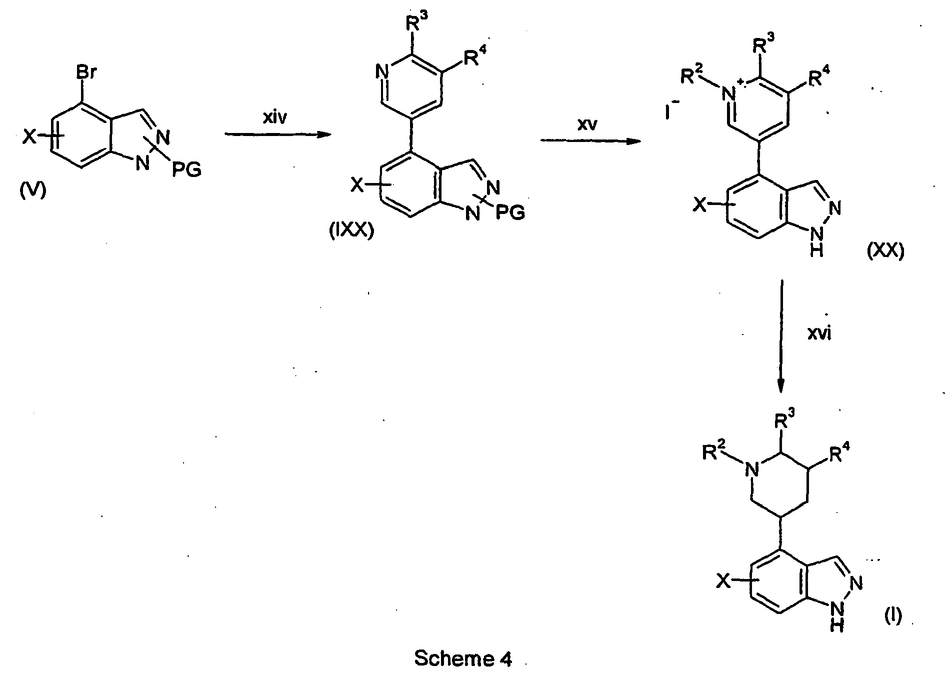

Compounds of formula (IXX) may be prepared from compounds of formula (V) by xiv) reaction with 3-pyridyl boranes (or similar boronic acid) in the presence of a suitable base and suitable palladium catalyst. Typical conditions comprise addition of the 3-pyridyl borane to a compound of formula (V) in toluene/ethanol as solvent, in the presence of tetrakis(triphenylphosphine)palladium(0) and sodium carbonate, followed by heating to reflux. Examples of 3-pyridyl boranes (or similar boronic acids) are commercially available.

Compounds of formula (XX) may be prepared from compounds of formula (IXX) by xv) addition of an alkyl iodide. Typical conditions comprise addition of the alkyl iodide to a compound of formula (IXX), in a suitable solvent such as acetonitrile and then heating to reflux.

Compounds of formula (I) may be prepared from compounds of formula (XX) by xvi) hydrogenation. Typical conditions comprise hydrogenation of a compound of formula (XX), at elevated pressure, in a suitable solvent such as ethanol, in the presence of a suitable catalyst such as PtO₂.

All of the above reactions and the preparations of novel starting materials using in the preceding methods are conventional and appropriate reagents and reaction conditions for their performance or preparation as well as procedures for isolating the desired products will be well-known to those skilled in the art with reference to literature precedents and the Examples and Preparations hereto.

The compounds of the present invention have utility as selective D3 agonists in the treatment of disease states. There are a number of compounds with activity as both D2 and D3 agonists; however the use of such compounds is associated with a large number of side effects including nausea, emesis, syncope, hypotension and bradycardia, some of which are a cause for serious concern.

It was previously held that the efficacy of the prior art compounds stemmed from their ability to agonise D2; however D2 agonism is implicated as a cause of the side effects detailed above.

The present invention provides a class of selective D3 agonists. Serendipitously, these have been found to be efficacious, whilst reducing the side effects associated with unselective prior art compounds.

Accordingly a further aspect of the invention provides a compound of formula (I) for use as a medicament.

Compounds of present invention are particularly useful in treating sexual dysfunction, female sexual dysfunction, including hypoactive sexual desire disorder, female sexual arousal disorder, female orgasmic disorder and sexual pain disorder, male erectile dysfunction, hypertension, neurodegeneration, depression, and psychiatric disorders.

Accordingly, the present invention provides for the use of a compound of formula (I) in the manufacture of a medicament for the treatment of sexual dysfunction.

The compounds of the present invention are useful in the treatment of male sexual dysfunction, particularly male erectile dysfunction. Male erectile dysfunction (MED), otherwise known as male erectile disorder, is defined as:
*"the inability to achieve and*/*or maintain a penile erection for satisfactory sexual performance" (NIH Consensus Development Panel* on *Impotence, 1993)"*

It has been estimated that the prevalence of erectile dysfunction (ED) of all degrees (minimal, moderate and complete impotence) is 52% in men 40 to 70 years old, with higher rates in those older than 70 (Melman et al 1999, J. Urology, 161, p5-11). The condition has a significant negative impact on the quality of life of the individual and their partner, often resulting in increased anxiety and tension which leads to depression and low self-esteem. Whereas two decades ago, MED was primarily considered to be a psychological disorder (Benet et al 1994 Comp. Ther., 20: 669-673), it is now known that for the majority of individuals there is an underlying organic cause. As a result, much progress has been made in identifying the mechanism of normal penile erection and the pathophysiologies of MED.

Penile erection is a haemodynamic event which is dependent upon the balance of contraction and relaxation of the corpus cavernosal smooth muscle and vasculature of the penis (Lemer et al 1993, J. Urology, 149, 1256-1255). Corpus cavernosal smooth muscle is also referred to herein as corporal smooth muscle or in the plural sense corpus cavemosa. Relaxation of the corpus cavernosal smooth muscle leads to an increased blood flow into the trabecular spaces of the corpus cavernosa, causing them to expand against the surrounding tunica and compress the draining veins. This produces a vast elevation in blood pressure which results in an erection (Naylor, 1998, Br. J. Urology, 81, 424-431).

The changes that occur during the erectile process are complex and require a high degree of co-ordinated control involving the peripheral and central nervous systems, and the endocrine system (Naylor, 1998, Br. J. Urology, 81, 424-431). Corporal smooth muscle contraction is modulated by sympathetic noradrenergic innervation via activation of postsynaptic α₁ adrenoceptors. MED may be associated with an increase in the endogenous smooth muscle tone of the corpus cavemosum. However, the process of corporal smooth muscle relaxation is mediated partly by non-adrenergic, non-cholinergic (NANC) neurotransmission. There are a number of other NANC neurotransmitters found in the penis, other than NO, such as calcitonin gene related peptide (CGRP) and vasoactive intestinal peptide (VIP). The main relaxing factor responsible for mediating this relaxation is nitric oxide (NO), which is synthesised from L-arginine by nitric oxide synthase (NOS) (Taub et al 1993 Urology, 42, 698-704). It is thought that reducing corporal smooth muscle tone may aid NO to induce relaxation of the corpus cavemosum. During sexual arousal in the male, NO is released from neurones and the endothelium and binds to and activates soluble guanylate cyclase (sGC) located in the smooth muscle cells and endothelium, leading to an elevation in intracellular cyclic guanosine 3',5'-monophosphate (cGMP) levels. This rise in cGMP leads to a relaxation of the corpus cavemosum due to a reduction in the intracellular calcium concentration ([Ca²⁺]ᵢ), via unknown mechanisms thought to involve protein kinase G activation (possibly due to activation of Ca²⁺ pumps and Ca²⁺-activated K⁺ channels).

Multiple potential sites have been identified within the central nervous system for the modulation of sexual behaviour. The key neurotransmitters are thought to be serotonin, norepinephrine, oxytocin, nitric oxide and dopamine. By mimicking the actions of one of these key neurotransmitters sexual function may be adjusted. Dopamine D3 receptors are expressed almost exclusively in the limbic area of the brain, regions involved in the reward, emotional and cognitive processes.

Without being bound by any theory, it appears that "due to its role in the control of locomotor activity, the integrity of the nigrostriatal dopaminergic pathway is also essential for the display of copulatory behaviour. Somehow, more specific to sexual function, it is likely that dopamine can trigger penile erection by acting on oxytocinergic neurons located in the paraventricular nucleus of the hypothalamus, and perhaps on the pro-erectile sacral parasympathetic nucleus within the spinal cord". It now appears that the significant site is D3 and not as previously thought, D2.

In essence, D3 is an initiator of sexual behaviour.

Accordingly, the present invention provides for, the use of a compound of formula (I) in the preparation of a medicament for the treatment or prevention of erectile dysfunction.

Patients with mild to moderate MED should benefit from treatment with the compounds according to the present invention, and patients with severe MED may also respond. However, early investigations suggest that the responder rate of patients with mild, moderate and severe MED may be greater with a selective D3 agonist/PDE5 inhibitor combination. Mild, moderate and severe MED will be terms known to the man skilled in the art, but guidance can be found in The Journal of Urology, vol. 151, 54-61 (Jan 1994).

Early investigations suggest the below mentioned MED patient groups should benefit from treatment with a selective D3 agonist and a PDE5i (or other combination set out hereinafter). These patient groups, which are described in more detail in Clinical Andrology vol. 23, no.4, p773-782 and chapter 3 of the book by I. Eardley and K. Sethia "Erectile Dysfunction-Current Investigation and Management, published by Mosby-Wolfe, are as follows: psychogenic, organic, vascular, endocrinologic, neurogenic, arteriogenic, drug-induced sexual dysfunction (lactogenic) and sexual dysfunction related to cavernosal factors, particularly venogenic causes.

Accordingly the present invention provides for the use of a compound of formula (I) in the preparation of a medicament in combination with a PDE5 inhibitor for the treatment of erectile dysfunction.

Suitable PDE5 inhibitors are described herein.

The compounds of the present invention are useful in the treatment or prevention of female sexual dysfunction (FSD), particularly female sexual arousal disorder (FSAD), hypoactive sexual desire disorder (HSDD; lack of interest in sex), FSAD with concomitant HSDD, and female orgasmic disorder (FOD; inability to achieve orgasm).

In accordance with the invention, FSD can be defined as the difficulty or inability of a woman to find satisfaction in sexual expression. FSD is a collective term for several diverse female sexual disorders (Leiblum, S.R. (1998) - Definition and classification of female sexual disorders. lnt. J. Impotence Res., 10, S104-S106; Berman, J.R., Berman, L. & Goldstein, I. (1999) - Female sexual dysfunction: Incidence, pathophysiology, evaluations and treatment options. Urology, 54, 385-391.). The woman may have lack of desire, difficulty with arousal or orgasm, pain with intercourse or a combination of these problems. Several types of disease, medications, injuries or psychological problems can cause FSD. Treatments in development are targeted to treat specific subtypes of FSD, predominantly desire and arousal disorders.

The categories of FSD are best defined by contrasting them to the phases of normal female sexual response: desire, arousal and orgasm (Leiblum, S.R. (1998) - Definition and classification of female sexual disorders. lnt. J. Impotence Res., 10, S104-S106). Desire or libido is the drive for sexual expression. Its manifestations often include sexual thoughts either when in the company of an interested partner or when exposed to other erotic stimuli. Arousal is the vascular response to sexual stimulation, an important component of which is genital engorgement and includes increased vaginal lubrication, elongation of the vagina and increased genital sensation/sensitivity. Orgasm is the release of sexual tension that has culminated during arousal.

Hence, FSD occurs when a woman has an inadequate or unsatisfactory response in any of these phases, usually desire, arousal or orgasm. FSD categories include hypoactive sexual desire disorder, sexual arousal disorder, orgasmic disorders and sexual pain disorders. Although the compounds of the invention will improve the genital response to sexual stimulation (as in female sexual arousal disorder), in doing so it may also improve the associated pain, distress and discomfort associated with intercourse and so treat other female sexual disorders.

Hypoactive sexual desire disorder is present if a woman has no or little desire to be sexual, and has no or few sexual thoughts or fantasies. This type of FSD can be caused by low testosterone levels, due either to natural menopause or to surgical menopause. Other causes include illness, medications, fatigue, depression and anxiety.

Female sexual arousal disorder (FSAD) is characterised by inadequate genital response to sexual stimulation. The genitalia do not undergo the engorgement that characterises normal sexual arousal. The vaginal walls are poorly lubricated, so that intercourse is painful. Orgasms may be impeded. Arousal disorder can be caused by reduced oestrogen at menopause or after childbirth and during lactation, as well as by illnesses, with vascular components such as diabetes and atherosclerosis. Other causes result from treatment with diuretics, antihistamines, antidepressants e.g. selective serotonin re-uptake inhibitors (SSRis) or antihypertensive agents.

Sexual pain disorders (includes dyspareunia and vaginismus) is characterised by pain resulting from penetration and may be caused by medications which reduce lubrication, endometriosis, pelvic inflammatory disease, inflammatory bowel disease or urinary tract problems.

As previously discussed, D3 is thought to be an initiator of sexual behaviour. The clitoris is considered to be a homologue of the penis (Levin, R.J. (1991), Exp. Clin. Endocrinol., 98, 61-69); the same mechanism that provides provides an erectile response in the male produces an increase in genital blood flow in the female with an associated effect upon FSD. In addition there are changes in proceptivity and receptivity.

Thus, in accordance with a preferred aspect of the invention, there is provided use of a compound of formula (I) in the preparation of a medicament for the treatment or prophylaxis of female sexual dysfunction, more particularly hypoactive sexual desire disorder, female sexual arousal disorder, female orgasmic disorder and sexual pain disorder.

Preferably the compounds of formula (I) are useful in the treatment or prophylaxis of female sexual arousal disorder (FSAD), FSAD with concomitant hypoactive sexual desire disorder, orgasmic disorder, and hypoactive sexual desire disorder, and most preferably in the treatment or prophylaxis of female sexual arousal disorder.

In a preferred embodiment the compounds of formula (I) are useful in the treatment of a subject with female sexual arousal disorder and concomitant hypoactive sexual desire disorder.

The Diagnostic and Statistical Manual (DSM) IV of the American Psychiatric Association defines Female Sexual Arousal Disorder (FSAD) as being:

*"... a persistent or recurrent inability to attain or* to *maintain until completion of the sexual activity adequate lubrication-swelling response of sexual excitement. The disturbance must cause marked distress or interpersonal difficulty....* ".

The arousal response consists of vasocongestion in the pelvis, vaginal lubrication and expansion and swelling of the external genitalia. The disturbance causes marked distress and/or interpersonal difficulty.

FSAD is a highly prevalent sexual disorder affecting pre-, peri- and post-menopausal (± hormone replacement therapy (HRT)) women. It is associated with concomitant disorders such as depression, cardiovascular diseases, diabetes and urogenital (UG) disorders.

The primary consequences of FSAD are lack of engorgement/swelling, lack of lubrication and lack of pleasurable genital sensation. The secondary consequences of FSAD are reduced sexual desire, pain during intercourse and difficulty in achieving an orgasm.

It has recently been hypothesised that there is a vascular basis for at least a proportion of patients with symptoms of FSAD (Goldstein et al., Int. J. lmpot. Res., 10, S84-S90,1998) with animal data supporting this view (Park et al., Int. J. Impot. Res., 9, 27-37, 1997).

R.J. Levin teaches us that because "... *male and female genitalia develop embryologically from the common tissue anlagen, [that] male and female genital structures are argued* to *be homologues* of *one another. Thus the clitoris is the penile homologue and the labia homologues* of *the scrotal sac...."* (Levin, R.J. (1991), Exp. Clin. Endocrinol., 98, 61-69).

Drug candidates for treating FSAD, which are under investigation for efficacy, are primarily erectile dysfunction therapies that promote circulation to male genitalia.

The compounds of the present invention are advantageous by providing a means for restoring a normal sexual arousal response - namely increased genital blood flow leading to vaginal, clitoral and labial engorgement. This will result in increased vaginal lubrication via plasma transudation, increased vaginal compliance and increased genital sensitivity. Hence, the present invention provides a means to restore, or potentiate, the normal sexual arousal response.

Thus, in accordance with a preferred aspect of the invention, there is provided use of a compound of formula (I) in the preparation of a medicament for the treatment or prophylaxis of female sexual arousal disorder and female sexual arousal disorder with concomitant hypoactive sexual desire disorder.

By female genitalia herein we mean: "The genital organs consist of an internal and external group. The internal organs are situated within the pelvis and consist of ovaries, the uterine tubes, uterus and the vagina. The external organs are superficial to the urogenital diaphragm and below the pelvic arch. They comprise the mons pubis, the labia majora and minora pudendi, the clitoris, the vestibule, the bulb of the vestibule, and the greater vestibular glands" (Gray's Anatomy, C.D. Clemente, 13^{th} American Edition).

The compounds of the invention find application in the following sub-populations of patients with FSD: the young, the elderly, pre-menopausal, peri-menopausal, post-menopausal women with or without hormone replacement therapy.

The compounds of the invention find application in patients with FSD arising from:-
i) Vasculogenic etiologies e.g. cardiovascular or atherosclerotic diseases, hypercholesterolemia, cigarette smoking, diabetes, hypertension, radiation and perineal trauma, traumatic injury to the iliohypogastric pudendal vascular system.
ii) Neurogenic etiologies such as spinal cord injuries or diseases of the central nervous system including multiple sclerosis, diabetes, Parkinsonism, cerebrovascular accidents, peripheral neuropathies, trauma or radical pelvic surgery.
iii) Hormonal/endocrine etiologies such as dysfunction of the hypothalamic/pituitary/gonadal axis, or dysfunction of the ovaries, dysfunction of the pancreas, surgical or medical castration, androgen deficiency, high circulating levels of prolactin e.g. hyperprolactinemia, natural menopause, premature ovarian failure, hyper and hypothyroidism.
iv) Psychogenic etiologies such as depression, obsessive compulsive disorder, anxiety disorder, postnatal depression/"Baby Blues", emotional and relational issues, performance anxiety, marital discord, dysfunctional attitudes, sexual phobias, religious inhibition or traumatic past experiences.
v) Drug-induced sexual dysfunction resulting from therapy with selective serotonin reuptake inhibitors (SSRis) and other antidepressant therapies (tricyclics and major tranquillizers), antihypertensive therapies, sympatholytic drugs, chronic oral contraceptive pill therapy.

The Compounds of the present invention are also useful in the treatment of depression.

Dopamine D3 receptors are expressed almost exclusively in the limbic area of the brain, regions involved in reward, emotional and cognitive processes. Chronic treatment with several classes of antidepressants are known to increase the expression of D3 in the limbic area, and antidepressant effects of desipramine can be blocked by sulpride (D2/D3 antagonist) when injected to nucleus accumbens (area rich in D3) but not caudate-putamen (area rich in dopamine D2 receptors). In addition, antidepressant effects were observed preclinical models of depression and in patients treated with pramipexole, a D3-preferring D2/D3 agonist. The available information suggests that D3 receptors mediate the anti-depressant activity and that selective D3 receptor agonists represent a new class of antidepressant drugs. Since antidepressants are known to be effective in other psychiatric disorders, D3 agonists would have the potential to treat psychiatric diseases.

Suitable conditions include depression (e.g. depression in cancer patients, depression in Parkinson's patients, postmyocardial infarction depression, subsyndromal symptomatic depression, depression in infertile women, major depression, child abuse induced depression, post partum depression and grumpy old man syndrome), single episodic or recurrent major depressive disorders, dysthymic disorders, depressive neurosis and neurotic depression, melancholic depression including anorexia, weight loss, insomnia, early morning waking or psychomotor retardation; atypical depression (or reactive depression) including increased appetite, hypersomnia, psychomotor agitation or irritability, seasonal affective disorder and pediatric depression; bipolar disorders or manic depression, for example, bipolar I disorder, bipolar II disorder and cyclothymic disorder; conduct disorder, disruptive behavior disorder, trichotillomania, kleptomania, attention deficit hyperactivity disorder (ADHD); behavioral disturbances associated with mental retardation, autistic disorder; borderline personality disorder, avoidant personality disorder, anxiety disorders such as panic disorder with or without agoraphobia, agoraphobia without history of panic disorder, specific phobias, for example, specific animal phobias, social anxiety, social phobia, obsessive-compulsive disorder, stress disorders including post-traumatic stress disorder and acute stress disorder, and generalized anxiety disorders; emotional lability, pathological crying; schizophrenia and other psychotic disorders, for example, schizophreniform disorders, schizoaffective disorders, delusional disorders, brief psychotic disorders, shared psychotic disorders, psychotic disorders with delusions or hallucinations, psychotic episodes of anxiety, anxiety associated with psychosis, psychotic mood disorders such as severe major depressive disorder; mood disorders associated with psychotic disorders such as acute mania and depression associated with bipolar disorder; mood disorders associated with schizophrenia; eating disorders (e.g. anorexia nervosa and bulimia nervosa), obesity; movement disorders such as akinesias, dyskinesias, including familial paroxysmal dyskinesias, spasticities, Tourette's syndrome, Scott syndrome, PALSYS and akinetic-rigid syndrome; extra-pyramidal movement disorders such as medication-induced movement disorders, for example, neuroleptic-induced Parkinsonism, neuroleptic malignant syndrome, neuroleptic-induced acute dystonia, neuroleptic-induced acute akathisia, neuroleptic-induced tardive dyskinesia and medicafion-induced postural tremour; chemical dependencies and addictions (e.g., dependencies on, or addictions to, alcohol, heroin, cocaine, benzodiazepines, nicotine, or phenobarbitol) and behavioral addictions such as an addiction to gambling; ocular disorders such as glaucoma and ischemic retinopathy; sleeping disorder (cataplexy) and shock.

In a preferred embodiment, the present invention provides for the use of a compound of formula (I) in the preparation of a medicament for the treatment of depression or psychiatric disorders.

Suitable depressive conditions and psychiatric disorders are described above.

In an additional further embodiment, the invention provides for the use of compounds of formula I in the preparation of a medicament for the treatment of obesity.

The compounds of the present invention also have utility in the treatment of neurodegeneration; sources of neurodegeneration include neurotoxin poisoning; vision loss caused by neurodegeneration of the visual pathway, such as by a stroke in the visual pathway eg in retina, optic nerve and/or occipital lobe; epileptic seizures; and from impairment of glucose and/or oxygen supply to the brain.

Conditions related to neurodegeneration include Restless Leg Syndrome, Huntington's disease, Multiple Sclerosis, mild cognitive impairment, Down's syndrome, stroke, Hereditary Cerebral Hemorrhage with Amyloidosis of the Dutch-Type, cerebral amyloid angiopathy, delirium, dementia, age-related cognitive decline (ARCD), and amnestic and other cognitive or neurodegenerative disorders, such as Parkinson's disease (PD), Huntington's disease (HD), Alzheimer's disease, senile dementia, dementia of the Alzheimer's type, memory disorders, loss of executive function, vascular dementia, dementias of mixed vascular and degenerative origin, dementia associated with Parkinson's disease, dementia associated with progressive supranuclear palsy, dementia associated with cortical basal degeneration, multi-infarct dementia, alcoholic dementia or other drug-related dementia, dementia associated with intracranial tumors or cerebral trauma, dementia associated with Huntington's disease, Pick's disease, Creutzfeldt-Jakob disease, HIV or AIDS-related dementia, diffuse Lewy body type of Alzheimer's disease, frontotemporal dementias with parkinsonism (FTDP), head trauma, spinal cord injury, demyelinating diseases of the nervous system, peripheral neuropathy, pain, cerebral amyloid angiopathy, amyotrophic lateral sclerosis, multiple sclerosis, dyskinesia associated with dopamine agonist therapy, mental retardation, learning disorders, including reading disorder, mathematics disorder, or a disorder of written expression; age-related cognitive decline, amnesic disorders, neuroleptic-induced parkinsonism, tardive dyskinesias, and acute and chronic neurodegenerative disorders.

Accordingly the present invention provides for the use of a compound of formula (I) in the preparation of a medicament for the treatment of neurodegeneration.

Suitable neurodegenerative conditions are described above.

In addition to their role in treating Sexual dysfunction, depression, neurodegeneration and psychiatric disorders, the compounds of the present invention are likely to be efficacious in a number of additional indications.

Accordingly, the present invention provides for the use of compounds of formula (I), in the preparation of a medicament for the treatment of hypertension, premature ejaculation, obesity, cluster headache, migraine, pain, endocrine disorders (e.g. hyperprolactinaemia), vasospasm (particularly in the cerebral vasculature), cerebellar ataxia, gastrointestinal tract disorders (involving changes in motility and secretion), premenstrual syndrome, fibromyalgia syndrome, stress incontinence, trichotillomania and chronic paroxysmal hemicrania, headache (associated with vascular disorders).

It is to be appreciated that all references herein to treatment include curative, palliative and prophylactic treatment.

### D3/D2 AGONIST ASSAY

Activity at the dopamine D3 receptor may be determined using the methods described in WO 2004/052372. Using this assay, the compounds of the present invention all exhibit a functional potency at D3 receptor expressed as an EC50, lower than 1000nM and a 10 fold selectivity for D3 over D2. Selectivity is calculated as the D2 EC50 value divided by the D3 EC50 value. Where the value of the D₂ EC50 was >10000, a figure of 10000 was used in the calculation.

The compound of example 13 has a functional potency at the D3 receptor, expressed as an EC₅₀, of 38 nM, with an Emax (maximal response value) of 74% (relative to the maximal effect of standard agent pramipexole). Against the D2 receptor this compound gave only a 28% repsonse (relative to the maximal effect of pramipexole) at 10000nM.

Suitable auxiliary active agents for use in the combinations of the present invention include:
1) Naturally occurring or synthetic prostaglandins or esters thereof. Suitable prostaglandins for use herein include compounds such as alprostadil, prostaglandin E_{1,}prostaglandin E₀, 13, 14 - dihydroprosta glandin E₁, prostaglandin E₂, eprostinol, natural synthetic and semi-synthetic prostaglandins and derivatives thereof including those described in WO-00033825 and/or US 6,037,346 issued on 14th March 2000 all incorporated herein by reference, PGE₀, PGE₁, PGA₁, PGB₁, PGF₁ α, 19-hydroxy PGA₁, 19-hydroxy - PGB₁, PGE₂, PGB₂, 19-hydroxy-PGA₂, 19-hydroxy-PGB₂, PGE₃α, carboprost tromethamine dinoprost, tromethamine, dinoprostone, lipo prost, gemeprost, metenoprost, sulprostune, tiaprost and moxisylate;
2) α - adrenergic receptor antagonist compounds also known as α - adrenoceptors or α-receptors or α-blockers. Suitable compounds for use herein include: the α-adrenergic receptor blockers as described in PCT application WO99/30697 published on 14th June 1998, the disclosures of which relating to α-adrenergic receptors are incorporated herein by reference and include, selective α₁-adrenoceptor or α₂-adrenoceptor blockers and non-selective adrenoceptor blockers, suitable α₁-adrenoceptor blockers include: phentolamine, phentolamine mesylate, trazodone, alfuzosin, indoramin, naftopidil, tamsulosin, dapiprazole, phenoxybenzamine, idazoxan, efaraxan, yohimbine, rauwolfa alkaloids, Recordati 15/2739, SNAP 1069, SNAP 5089, RS17053, SL 89.0591, doxazosin, terazosin, abanoquil and prazosin; α₂-blocker blockers from US 6,037,346 [14th March 2000] dibenarnine, tolazoline, trimazosin and dibenamine; α-adrenergic receptors as described in US patents: 4,188,390; 4,026,894; 3,511,836; 4,315,007; 3,527,761; 3,997,666; 2,503,059; 4,703,063; 3,381,009; 4,252,721 and 2,599,000 each of which is incorporated herein by reference; α₂-Adrenoceptor blockers include: clonidine, papaverine, papaverine hydrochloride, optionally in the presence of a cariotonic agent such as pirxamine;
3) NO-donor (NO-agonist) compounds. Suitable NO-donor compounds for use herein include organic nitrates, such as mono- di or tri-nitrates or organic nitrate esters including glyceryl trinitrate (also known as nitroglycerin), isosorbide 5-mononitrate, isosorbide dinitrate, pentaerythritol tetranitrate, erythrityl tetranitrate, sodium nitroprusside (SNP), 3-morpholinosydnonimine molsidomine, S-nitroso- N-acetyl penicilliamine (SNAP) S-nitroso-N-glutathione (SNO-GLU), N-hydroxy - L-arginine, amylnitrate, linsidomine, linsidomine chlorohydrate, (SIN-1) S-nitroso - N-cysteine, diazenium diolates,(NONOates), 1,5-pentanedinitrate, L-arginene, ginseng, zizphi fructus, molsidomine, Re - 2047, nitrosylated maxisylyte derivatives such as NMI-678-11 and NMI-937 as described in published PCT application WO 0012075;
4) Potassium channel openers or modulators. Suitable potassium channel openers/modulators for use herein include nicorandil, cromokalim, levcromakalim, lemakalim, pinacidil, cliazoxide, minoxidil, charybdotoxin, glyburide, 4-amini pyridine, BaCl₂;
5) Vasodilator agents. Suitable vasodilator agents for use herein include nimodepine, pinacidil, cyclandelate, isoxsuprine, chloroprumazine, , Rec 15/2739, trazodone;
6) Thromboxane A2 agonists;
7) CNS active agents;
8) Ergot alkoloids; Suitable ergot alkaloids are described in US patent 6,037,346 issued on 14th March 2000 and include acetergamine, brazergoline, bromerguride, cianergoline, delorgotrile, disulergine, ergonovine maleate, ergotamine tartrate, etisulergine, lergotrile, lysergide, mesulergine, metergoline, metergotamine, nicergoline, pergolide, propisergide, proterguride and terguride;
9) Compounds which modulate the action of natruretic factors in particular atrial naturetic factor (also known as atrial naturetic peptide), B type and C type naturetic factors such as inhibitors or neutral endopeptidase;
10) Compounds which inhibit angiotensin-converting enzyme such as enapril, and combined inhibitors of angiotensin-converting enzyme and neutral endopeptidase such as omapatrilat.
11) Angiotensin receptor antagonists such as losartan;
12) Substrates for NO-synthase, such as L-arginine;
13) Calcium channel blockers such as amlodipine;
14) Antagonists of endothelin receptors and inhibitors or endothelin-converting enzyme;
15) Cholesterol lowering agents such as statins (e.g. atorvastatin/ Lipitor- trade mark) and fibrates;
16) Antiplatelet and antithrombotic agents, e.g. tPA, uPA, warfarin, hirudin and other thrombin inhibitors, heparin, thromboplastin activating factor inhibitors;
17) Insulin sensitising agents such as rezulin and hypoglycaemic agents such as glipizide;
18) Acetylcholinesterase inhibitors such as donezipil;
19) Steroidal or non-steroidal anti-inflammatory agents;
20) Estrogen receptor modulators and/or estrogen agonists and/or estrogen antagonists, preferably raloxifene or lasofoxifene, (-)-cis-6-phenyl-5-[4-(2-pyrrolidin-1-yl-ethoxy)-phenyl]-5,6,7,8-tetrahydronaphthalene-2-ol and pharmaceutically acceptable salts thereof the preparation of which is detailed in WO 96/21656;
21) A PDE inhibitor, more particularly a PDE 2, 3, 4, 5, 7 or 8 inhibitor, preferably PDE2 or PDE5 inhibitor and most preferably a PDE5 inhibitor (see hereinafter), said inhibitors preferably having an IC50 against the respective enzyme of less than 100nM (with the proviso that PDE 3 and 4 inhibitors are only administered topically or by injection to the penis);
22) Vasoactive intestinal protein (VIP), VIP mimetic, VIP analogue, more particularly mediated by one or more of the VIP receptor subtypes VPAC1,VPAC or PACAP (pituitory adenylate cyclase activating peptide), one or more of a VIP receptor agonist or a VIP analogue (e.g. Ro-125-1553) or a VIP fragment, one or more of a α-adrenoceptor antagonist with VIP combination (e.g. Invicorp, Aviptadil);
23) A melanocortin receptor (particularly of the MC3 or MC4 subtype) agonist or modulator or melanocortin enhance, such as melanotan II, PT-14, PT-141 or compounds claimed in WO-09964002, WO-00074679, WO-09955679, WO-00105401, WO-00058361, WO-00114879, WO-00113112, WO-09954358;
24) A serotonin receptor agonist, antagonist or modulator, more particularly agonists, antagonists or modulators for 5HT1A (including VML 670), 5HT2A, 5HT2C, 5HT3 and/or 5HT6 receptors, including those described in WO-09902159, WO-00002550 and/or WO-00028993;
25) A testosterone replacement agent (including dehydroandrostendione), testosternone (Tostrelle), dihydrotestosterone or a testosterone implant;
26) Estrogen, estrogen and medroxyprogesterone or medroxyprogesterone acetate (MPA) (i.e. as a combination), or estrogen and methyl testosterone hormone replacement therapy agent (e.g. HRT especially Premarin, Cenestin, Oestrofeminal, Equin, Estrace, Estrofem, Elleste Solo, Estring, Eastraderm TTS, Eastraderm Matrix, Dermestril, Premphase, Preempro, Prempak, Premique, Estratest, Estratest HS, Tibolone);
27) A modulator of transporters for noradrenaline, dopamine and/or serotonin, such as bupropion, GW-320659;
28) A purinergic receptor agonist and/or modulator,
29) A neurokinin (NK) receptor antagonist, including those described in WO-09964008;
30) An opioid receptor agonist, antagonist or modulator, preferably agonists for the ORL-1 receptor,
31) An agonist, antagonist or modulator for oxytocin receptors, preferably a selective oxytocin agonist or modulator;
32) Modulators of cannabinoid receptors;
33) A SEP inhibitor (SEPi), for instance a SEPi having an IC₅₀ at less than 100 nanomolar, more preferably, at less than 50 nanomolar.
   Preferably, the SEP inhibitors according to the present invention have greater than 30-fold, more preferably greater than 50-fold selectivity for SEP over neutral endopeptidase NEP EC 3.4.24.11 and angiotensin converting enzyme (ACE). Preferably the SEPi also has a greater than 100-fold selectivity over endothelin converting enzyme (ECE).
34) An antagonist or modulator for the NPY (particularly Y1 and Y5 subtype) receptor.
35) A Sex Hormone Binding Globulin antagonist or modulator that inhibits estrogens and/or androgens from being bound.
36) An arginase II inhibitor,
37) An agonist, antagonist or modulator for vassopressin receptors, preferably selective for the V1a receptor
38) A PDE5 Inhibitor. Suitable PDE5 inhibitors include:
   5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil), particularly sildenafil citrate;
   (6R,12aR)-2,3,6,7,12,12a-hexahydro-2-methyl-6-(3,4-methylenedioxyphenyl) - pyrazino[2',1':6,1]pyndo[3,4-b]indole-1,4-dione (IC-351 or tadalafil);
   2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil); 5-(5-Acetyl-2-butoxy-3-pyridinyl)-3-ethyl-2-(1-ethyl-3-azetidinyl)-2,6-dihydro-7*H*-pyrazolo[4,3-*d*]pyrimidin-7-one; 5-(5-Acetyl-2-propoxy-3-pyridinyl)-3-ethyl-2-(1-isopropyl-3-azetidinyl)-2,6-dihydro-7*H*-pyrazolo[4,3-*d*]pyrimidin-7-one ; 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one; 4-[(3-chloro-4-methoxybenzyl)amino]-2-[(2S)-2-(hydroxymethyl)pyrrolidin-1-yl]-N-(pyrimidin-2-ylmethyl)pyrimidine-5-carboxamide (TA-1790); 3-(1-methyl-7-oxo-3-propyl-6,7-dihydro-1H-pyrazolo[4,3-d]pyrimidin-5-yl)-N-[2-(1-methylpyrrolidin-2-yl)ethyl]-4-propoxybenzenesulfonamide (DA 8159) and pharmaceutically acceptable salts thereof.
39) A selective dopamine D4 receptor agonist such as 2-[(4-pyridin-2-yipiperazin-1-yl)methyl]-1H-benzimidazole (ABT724).

By cross reference herein to compounds contained in patents and patent applications which can be used in accordance with invention, we mean the therapeutically active compounds as defined in the claims (in particular of claim 1) and the specific examples (all of which is incorporated herein by reference).

If a combination of active agents is administered, then they may be administered simultaneously, separately or sequentially. ,

The compounds of formula I should be assessed for their biopharmaceutical properties, such as solubility and solution stability (across pH), permeability, etc., in order to select the most appropriate dosage form and route of administration for treatment of the proposed indication.

Compounds of the invention intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

They may be administered alone or in combination with one or more other compounds of the invention or in combination with one or more other drugs (or as any combination thereof). Generally, they will be administered as a formulation in association with one or more pharmaceutically acceptable excipients. The term 'excipient' is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in Remington's Pharmaceutical Sciences, 19th Edition (Mack Publishing Company, 1995).

Accordingly the present invention provides for a composition comprising a compound of formula (I), and a pharmaceutically acceptable diluent or carrier.

The compounds of the invention may be administered orally. Oral administration may involve swallowing, so that the compound enters the gastrointestinal tract, and/or buccal, lingual, or sublingual administration
by which the compound enters the blood stream directly from the mouth.

Formulations suitable for oral administration include solid, semi-solid and liquid systems such as tablets; soft or hard capsules containing multi- or nano-particulates, liquids, or powders; lozenges (including liquid-filled); chews; gels; fast dispersing dosage forms; films; ovules; sprays; and buccal/mucoadhesive patches.

Liquid formulations include suspensions, solutions, syrups and elixirs. Such formulations may be employed as fillers in soft or hard capsules (made, for example, from gelatin or hydroxypropylmethylcellulose) and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human or veterinary use are typically pliable water-soluble or water-swellable thin film dosage forms which may be rapidly dissolving or mucoadhesive and typically comprise a compound of formula I, a film-forming polymer, a binder, a solvent, a humectant, a plasticiser, a stabiliser or emulsifier, a viscosity-modifying agent and a solvent. Some components of the formulation may perform more than one function.

The compound of formula I may be water-soluble or insoluble. A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes. Alternatively, the compound of formula I may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line, 25(2), 1-14, by Verma et al (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular, intrasynovial and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula I used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a suspension or as a solid, semi-solid, or thixotropic liquid for administration as an implanted depot providing modified release of the active compound. Examples of such formulations include drug-coated stents and semi-solids and suspensions comprising drug-loaded poly(dl-lactic-coglycolic)acid (PGLA) microspheres.

The compounds of the invention may also be administered topically, (intra)dermally, or transdermally to the skin or mucosa. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Other means of topical administration include delivery by electroporation, iontophoresis, phonophoresis, sonophoresis and microneedle or needle-free (*e.g.* Powderject™, Bioject™, *etc.*) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler, as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane, or as nasal drops. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as *l*-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula l, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from ... to ... µg of the compound of formula 1. The overall daily dose will typically be in the range ... µg to ... mg which may be administered in a single dose or, more usually, as divided doses throughout the day.

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, gels, biodegradable (*e.g.* absorbable gel sponges, collagen) and non-biodegradable (*e.g.* silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for
example, gelan gum, may be incorporated together with a preservative, such as benzalkonium chloride. Such formulations may also be delivered by iontophoresis.
Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i.e.* as a carrier, diluent, or solubiliser. Most commonly used for these purposes are alpha-, beta- and gamma-cyclodextrins, examples of which may be found in international Patent Applications Nos. WO 91/11172, WO 94/02518 and WO 98/55148.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula I in accordance with the invention, and means for separately retaining said compositions, such as a container, divided bottle, or divided foil packet. An example of such a kit is the familiar blister pack used for the packaging of tablets, capsules and the like..

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

The invention is illustrated by the following non-limiting examples in which the following abbreviations and definitions are used:
- α_{D}: optical rotation at 587nm.
- Ac₂O: acetic anhydride
- APCl: atmospheric pressure chemical ionisation
- Arbacel®: filter agent
- br: broad
- Boc: *tert*-butoxycarbonyl
- Bu: butyl
- Celite: filter agent
- CDCl₃: chloroform-d1
- CD₃OD: methanol-d4
- δ: chemical shift
- d: doublet
- dd: double doublet
- DCM: dichloromethane
- DMF: *N,N*-dimethylformamide
- DMSO: dimethylsulfoxide
- eq: (molar) equivalents
- ESI: electrospray ionisation
- Et: ethyl
- EtOAc: ethyl acetate
- h/hr: hours
- HCl: hydrogen chloride
- HPLC: high performance liquid chromatography
- HR M/S: high resolution mass spectrum
- IPA: isopropylalcohol
- KOAc: potassium acetate
- m: multiplet
- Me: methyl
- MeCN: acetonitrile
- M/S: mass spectrum
- min: minutes
- NMR: nuclear magnetic resonance
- q: quartet
- r.t.: room temperature
- s: singlet
- sat: saturated
- t: triplet
- td: triplet of doublets
- Tf: trifluoromethanesulfonyl
- TFA: trifluoroacetic acid
- THF: tetrahydrofuran
- TIPS: triisopropylsilyl
- TLC/t.I.c: thin layer chromatography

¹H Nuclear magnetic resonance (NMR) spectra were in all cases consistent with the proposed structures. Characteristic chemical shifts (δ) are given in parts-per-million _{H} downfield from tetramethylsilane using conventional abbreviations for designation of major peaks: e.g. s, singlet; d, doublet; t, triplet; q, quartet; m, multiplet; br, broad. The following abbreviations have been used for common solvents: CDCl₃, deuterochloroform; DMSO, dimethylsulfoxide. The abbreviation psi means pounds per square inch and LRMS means low resolution mass spectrometry. Where thin layer chromatography (TLC) has been used it refers to silica gel TLC using silica gel 60 F₂₅₄ plates, R_{f} is the distance travelled by a compound divided by the distance travelled by the solvent front on a TLC plate.

### Examples 1 and 2

### 4-[(2R,5S)-5-methyl-4-propylmorpholin-2-yl]-1H-indazole & 4-[(2S,5S)-5-methyl-4-propylmorpholin-2-yl]-1H-indazole

Concentrated H₂SO₄ (9 mL) was added to (2*S*)-2-[[2-hydroxy-2-(2-trityl-2*H*-indazol-4-yl)ethyl](propyl)amino]propan-1-ol (660 mg, 1.3 mmol; preparation 7) in CH₂Cl₂ (12 mL) at room temperature and stirred at room temperature for 3 h. The reaction mixture was then basified to pH 8 by cautious addition of 0.880 NH₃, extracted with EtOAc (2 x 100 mL) and CH₂Cl₂ (100 mL). The combined organic layers were dried over MgSO₄, filtered and solvent evaporated. This material was purified by flash chromatography on a SiO₂ column with a gradient elution from 100% CH₂Cl₂ to 98/2/0.5 CH₂Cl₂/MeOH/0.880 NH₃ to give 230 mg of a mixture of diastereoisomers as a brown oil.
A sample of this mixture (150 mg) was separated by HPLC on a chiralpak AD-H column, at a flow rate of 15 mL/minute with a mobile phase of 50% MeOH/EtOH to give

### Diastereoisomer 1,

### 4-[(2R,5S)-5-methyl-4-propylmorpholin-2-yl]-1H-indazole

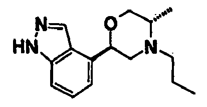

### 38 mg, retention time 4.8 min, clear oil

### M/S (ESI+) 260 (MH+)

¹H NMR (400MHz, CDCl₃) δ_{H} : 0.9 (t, 3H), 1.1 (d, 3H), 1.4-1.7 (m, 2H), 2.2-2.35 (m, 1H), 2.4 (m, 1H), 2.5-2.6 (m, 1H), 2.75-2.9 (m, 1H), 3.1 (m, 1H), 3.4-3.6 (m, 1H), 3.9-4.0 (m, 1H), 4.9-5.0 (m, 1H), 7.15 (d, 1H), 7.35 (t, 1H), 7.45 (d, 1H), 8.2 (s, 1 H)

### Diastereoisomer 2;

### 4-[(2S,5S)-5-methyl-4-propylmorpholin-2-yl]-1H-indazole

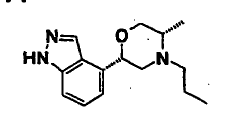

### 50 mg, retention time 6.6 min, white solid

### M/S (ESI+) 260 (MH+), 282 (MNa+)

¹H NMR (400MHz, CDCl₃) δ_{H} : 0.9 (t, 3H), 1.2 (d, 3H), 1.45-1.65 (m, 2H), 2.3-2.5 (m, 2H), 2.6-2.8 (m, 2H), 3.0 (m, 1H), 3.85-3.95 (m, 1 H), 4.0 (m, 1H), 4.9-5.0 (m, 1H), 7.15 (d, 1H), 7.35 (t, 1H), 7.45 (d, 1H), 8.2 (s, 1H)

### Examples 3 and 4

### 4-[(2R)-4-propylmorpholin-2-yl]-1H-indazole & 4-[(2S)-4-propylmorpholin-2-yl]-1H-indazole

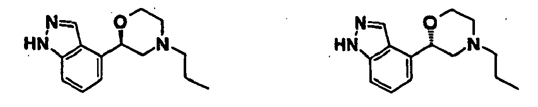

Concentrated H₂SO₄ (25 mL) was added to 2-[(2-hydroxyethyl)(propyl)amino]-1-(2-trityl-2*H*-indazol-4-yl)ethanol (1.10 g 2.17 mmol; preparation 9) in CH₂Cl₂ (20 mL) at r.t. and stirred for 1 h. The reaction mixture was cooled in an ice bath, and basified to pH 8 by cautious addition of 2.5N aqueous NaOH, extracted with EtOAc (3 x 100 mL). The combined organics combined, dried over MgSO₄, filtered and solvent evaporated. This material was purified by flash chromatography on a SiO₂ column eluting with 95/5/0.5 CH₂Cl₂/MeOH/0.880 NH₃ to give 300mg (48%) of the racemic mixture as a yellow glass.

The racemate was resolved on a Chiralpack AD column using 80:20 hexane : IPA with 0.1 % diethylamine modifier as mobile phase to give:
Enantiomer 1
   Retention time 7.3 min
   ¹H NMR (400MHz, d₆-DMSO) δ_{H} 0.85 (t, 3H), 1.35-1.55 (m, 2H), 2.0-2.4 (m, 4H), 2.7-3.0 (m, 2H), 3.65-3.8 (m, 1H), 3.9-4.0 (m, 1H), 4.8-4.9 (m, 1H), 7.05 (d, 1 H), 7.3 (t, 1H). 7.45 (d, 1H), 8.15 (s, 1H), 13.0-13.1 (br s, 1H)
   Elemental Analysis:
   C₁₄H₁₉N₃O.0.5.MeOH requires C 66.64%, H 8.10%, N 16.1%
   Found C 66.95%, H 8.4%, N 15.7%
   M/S (APCI+) 246 (MH+)
   HR M/S 246.1598
Enantiomer 2
   Retention time 13.0 min
   ¹H NMR (400MHz, d₆-DMSO) δ_{H} 0.85 (t, 3H), 1.35-1.55 (m, 2H), 2.0-2.4 (m, 4H), 2.7-3.0 (m, 2H), 3.65-3.8
   (m, 1H). 3.9-4.0 (m, 1 H), 4.8-4.9 (m, 1H). 7.05 (d, 1H). 7.3 (t, 1 H), 7.45 (d, 1H), 8.15 (s, 1H), 13.0-13.1 (br s, 1H)

### Example 5

### 4-azetidin-3-yl-1H-indazole hydrochloride

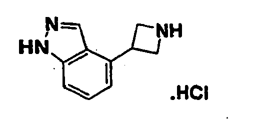

*tert*-butyl 3-(2-trityl-2*H*-indazol-4-yl)azetidine-1-carboxylate (500 mg, 0.9 mmol; preparation 10) treated with a solution of 1M HCl in Et₂O and stirred at r.t. for 18 h. The solvent was evaporated, and the resulting solid was triturated with Et₂O (3 x 50 mL) and the resulting solid dried *in vacuo* to give a pale orange solid of *title compound* (180 mg, 88%)
M/S (ESI+) 174 (MH+)
¹H NMR (400MHz, CD₃OD) δ_{H} 4.4-4.6 (m, 4H), 4.7-4.8 (m, 1H), 7.25 (d, 1H), 7.5-7.6 (m, 2H), 8.35 (s, 1H)

### Example 6

### 4-[1-(2-phenylethyl)azetidin-3-yl]-1H-indazole

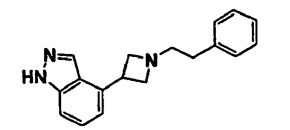

A portion of the crude mixture of 4-azetidin-3-yl-1*H*-indazole trifluoroacetate from preparation 11 (1 g, containing ca. 230 mg of desired azetidine, 1.3 mmol, 1 eq) was combined with phenylacetaldehyde (470 µL, 4.0 mmol, 3.1 eq), sodium triacetoxyborohydride (850 mg, 4 mmol, 3 eq) and CH₂Cl₂ (10 mL) and stirred at r.t. for 18 h. The solvent was evaporated and the crude material purified by flash chromatography on a SiO₂ column with an eluent of CH₂Cl₂/MeOH/0.880 NH₃ 95/5/0.5 to give 150 mg (41 %) of the *title compound* as a clear oil.
M/S (APCI+) 278 (MH+), 300 (MNa+)
M/S (APCl-) 276 (M-1)
¹H NMR (400MHz, CD₃OD) δ_{H} 2.7-2.9 (m, 4H), 3.3-3.45 (m, 2H), 3.9-4.0 (m, 2H), 4.1-4.25 (m, 1H), 7.0 (d, 1H), 7.15-7.45 (m, 7H), 8.05 (s, 1H).

### Example 7

### 4-(1-ethylazetidin-3-yl)-1H-indazole

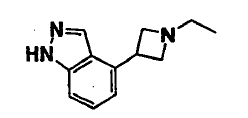

A portion of the crude mixture of 4-azetidin-3-yl-1*H*-indazole trifluoroacetate from preparation 11 (1 g, containing ca. 230 mg of desired azetidine, 1.3 mmol, 1 eq) was combined with acetaldehyde (230 µL, 4.0 mmol, 3.1 eq), sodium triacetoxyborohydride (850 mg, 4 mmol, 3.1 eq) and CH₂Cl₂ (10 mL) and stirred at r.t. for 18 h. The solvent was evaporated and the crude material purified by flash chromatography on a SiO₂ column with a gradient eluent of CH₂Cl₂/MeOH/0.880 NH₃ 97/3/0.5 to 95/5/0.5 to give 100 mg of partially purified *title compound* as a light brown oil. This was further purified by HPLC on an automated HPLC system:
Phenomenex Luna C18(2) column 150x15mm (10 micron particle size, 100Å porosity), using a 2 solvent eluent of acetonitrile : water : trifluoroacetic acid (5 : 95 : 0.1) [solvent A] and acetonitile [solvent B]. A solvent gradient is run at a flow-rate of 20 mL/min as in the table below.

| Time (min) | %B |
|---|---|
| 0 | 5 |
| 0.6 | 5 |
| 9.5 | 95 |
| 10.5 | 95 |
| 10.6 | 5 |
| 12 | 5 |

This afforded 90 mg of a clear oil of paritally purified *title compound,* which was further purified by flash chromatography on a SiO₂ column with an eluent of CH₂Cl₂/MeOH/0.880 NH₃ 90/10/1 to give 40mg(15%) of *title compound* a clear oil.
M/S (APCl+) 202 (MH+)
M/S (APCI-) 200 (M-1)
¹H NMR (400MHz, CD₃OD) δ_{H} 1.05 (t, 3H), 2.65 (q, 2H), 3.4 (m, 2H), 4.0 (m, 2H), 4.2 (m, 1H), 7.0 (d, 1H), 7.3-7.5 (m, 2H), 8.05 (s, 1H).

### Example 8

### 4-(1-isobutylazetidin-3-yl)-1H-indazole

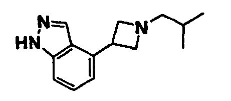

A portion of the crude mixture of 4-azetidin-3-yl-1*H*-indazole trifluoroacetate from preparation 11 (800 mg, containing ca. 180 mg of desired azetidine, 1.0 mmol, 1 eq) was combined with isobutyraldehyde (290 µL, 3.1 mmol, 3.1 eq), sodium triacetoxyborohydride (660 mg, 3.1 mmol, 3.1 eq) and CH₂Cl₂ (8 mL) and stirred at r.t. for 18 h. The solvent was evaporated and the crude material purified by flash chromatography on a SiO₂ column with an eluent of CH₂Cl₂/MeOH/0.880 NH₃ 96/4/0.5 to give 100 mg of partially purifed material as a brown oil.
This was further purified using the same automated HPLC system as in Example 7. The resulting material was further purified through a SiO₂ pad with an eluent of CH₂Cl₂/MeOH/0.880 NH₃ 90/10/1 to give 50mg (21 %) of the *title compound* as a white solid.
M/S (ESI+) 230 (MH+)
M/S (ESI-) 228 (M-1)
¹H NMR (400MHz, CD₃OD) δ_{H} 0.9 (d, 6H), 1.7-1.8 (m, 1H), 2.45 (d, 2H), 3.4 (m, 2H), 4.0 (m, 2H), 4.2 (m, 1H), 7.0 (d, 1H). 7.3-7.55 (m, 2H), 8.05 (s, 1 H).

### Example 9

### 4-(1-butylazetidin-3-yl)-1H-indazole

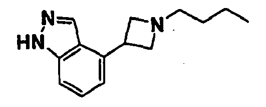

A portion of the crude mixture of 4-azetidin-3-yl-1*H*-indazole trifluoroacetate from preparation 11 (1 g, containing ca. 230 mg of desired azetidine, 1.3 mmol, 1 eq) was combined with butyraldehyde (360 µL, 4.0 mmol, 3.1eq), sodium triacetoxyborohydride (850 mg, 4 mmol, 3.1 eq) and CH₂Cl₂ (10 ml) and stirred at r.t. for 18 h. The solvent was evaporated and the crude material purified by flash chromatography on a SiO₂ column with a gradient eluent of CH₂Cl₂/MeOH/0.880 NH₃ 98/210.5 to 96/4/0.5 to give 100 mg of partially purified material as a light brown oil. This was further purified by using the same automated HPLC system as in Example 7. Further purification on a SiO₂ column with an eluent of CH₂Cl₂/MeOH/0.880 NH₃ 90/10/1 afforded 60 mg (20%) of *title compound* as a clear oil.
M/S (ESI+) 230 (MH+)
M/S (ESI-) 228 (M-1)
¹H NMR (400MHz, CD₃OD) δ_{H} 0.9 (t, 3H), 1.3-1.5 (m, 4H), 2.6 (t, 2H), 3.4 (m, 2H), 4.0 (m, 2H), 4.2 (m, 1H), 7.0 (d, 1H), 7.3-7.55 (m, 2H), 8.05 (s, 1H).

### Example 10

### 4-[1-(3-phenytpropyl)azetidin-3-yl]-1H-indazole

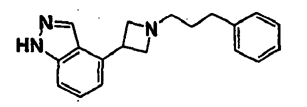

A portion of the crude mixture of 4-azetidin-3-yl-1*H*-indazole trifluoroacetate from preparation 11 (700 mg, containing ca. 161 mg of desired azetidine, 0.9 mmol, 1 eq) combined with 3-phenylpropionaldehyde (370 µL, 2.8 mmol, 3 eq), sodium triacetoxyborohydride (600 mg, 2.8 mmol, 3 eq) and CH₂Cl₂ (5 ml) and stirred at r.t. for 18 h. The solvent was evaporated and the crude material purified by flash chromatography on a SiO₂ column with an eluant of CH₂Cl₂/MeOH/0.880 NH₃ 97/3/0.5 to give 100mg (37%) of the *title compound* a light brown oil.
M/S (ESI+) 292 (MH+)
M/S (ESI-) 290 (M-1)
¹H NMR (400MHz, CD₃OD) δ_{H} 1.65-1.8 (m, 2H), 2.5-2.7 (m, 4H), 3.3-3.4 (m, 2H), 3.9-4.0 (m, 2H), 4.1-4.2 (m, 1H), 7.0 (d, 1H), 7.1-7.4 (m, 8H), 8.05 (s, 1H).

### Examples 11 and 12

### 4-{1-[(1S)-1-methylpropyl]azetidin-3-yl}-1H-indazole & 4-{1-[(1R)-1-methylpropyl]azetidin-3-yl}-1H-indazole

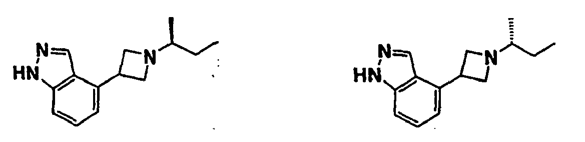

A portion of the crude mixture of 4-azetidin-3-yl-1*H*-indazole trifluoroacetate from preparation 11 (900 mg, containing ca. 210 mg of desired azetidine, 1.2 mmol, 1 eq) was combined with 2-butanone (330 µL, 3.6 mmol, 3 eq), sodium triacetoxyborohydride (760 mg, 3.6 mmol, 3 eq) and CH₂Cl₂ (5 mL) and stirred at r.t. for 18 h. The solvent was evaporated and the crude material purified by flash chromatography on a SiO₂ column with an eluent of CH₂Cl₂/MeOH/0.880 NH₃ 97/3/0.5 to give 130 mg of racemic mixture as a light brown oil.
This material was separated by HPLC on a Chiralpack AD-H column (250 x 21.2mm) with a MeOH:EtOH 50:50 mobile phase at a flow rate of 10 mL/min to give;
Enantiomer 1
25 mg clear oil
Retention time = 5.1 min
M/S (ESI+) 230 (MH+)
M/S (ESI-) 228 (M-1)
¹H NMR (400MHz. CD₃OD) δ_{H} 0.9 (t, 3H), 1.0 (d, 3H), 1.5-1.7 (m, 1H), 2.2-2.3 (m, 1H), 3.3-3.4 (m, 2H), 3.9-4.0 (m, 2H), 4.05-4.15 (m, 1H), 7.0 (d, 1 H), 7.3-7.45 (m, 2H), 8.05 (s, 1H).

Enantiomer 2
36 mg clear oil
Retention time = 5.8min
M/S (ESI+) 230 (MH+)
M/S (ESI-) 228 (M-1)
¹H NMR (400MHz, CD₃OD) δ_{H} 0.9 (t, 3H), 1.0(d, 3H), 1.5-1.7 (m, 1H), 2.2-2.3 (m, 1H), 3.3-3.4 (m, 2H), 3.9-4.0 (m, 2H), 4.05-4.15 (m, 1 H), 7.0 (d, 1H), 7.3-7.45 (m, 2H), 8.05 (s, 1 H).

### Example 13

### 4-(1-propylazetidin-3-yl)-1H-indazole

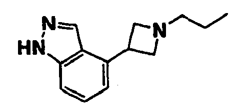

*tert*-butyl 3-(2-trityl-2*H*-indazol-4-yl)azetidine-1-carboxylate (3.45 g, 6.6 mmol; preparation 10), TFA (15 mL) and CH₂Cl₂ (20 mL) were stirred at r.t. overnight. The solvent was removed *in vacuo* to give a dark brown oil. This material was dissolved in CH₂Cl₂ and treated with propionaldehyde (974 µL, 13.4 mmol, 2 eq) and sodium triacetoxyborohydride (2.84 g, 13.4 mmol, 2 eq) and allowed to stir at r.t. for 4 h. The solvent was removed *in vacuo* and the material purified by flash chromatography on a SiO₂ column with an eluent of 97/3/0.5 CH₂Cl₂/MeOH/0.880 NH₃ to give 900mg of material with the trityl protecting group still attached. This material (900 mg, 1.9mmol) was dissolved in CH₂Cl₂ (5 mL), treated with TFA (4 mL) and Et₃SiH (470 µL, 2.9 mmol) and stirred at r.t. for 2 h. The solvent was evaporated and the material purified by flash chromatography on a SiO₂ column with an eluent of 96/4/0.5 EtOAc/MeOH/0.880 NH₃ to give 180 mg (13%) of the *title compound* a white solid.
M/S (ESI+) 216 (MH+)
M/S (ESI-) 214 (M-1)
¹H NMR (400MHz, CD₃OD) δ_{H} 0.95 (t, 3H), 1.4-1.6 (m, 2H), 2.55 (t, 2H), 3.3-3.4 (m, 2H), 3.9-4.0 (m, 2H), 4.1-4.25 (m, 1H), 7.0 (d, 2H), 7.3-7.5 (m, 2H), 8.05 (s, 1H).

### Examples 14 and 15

### 4-[(2R)-4-propylmorpholin-2-yl]-1,3-dihydro-2H-indol-2-one & 4-[(2S)-4-propylmorpholin-2-yl]-1,3-dihydro-2H-indol-2-one

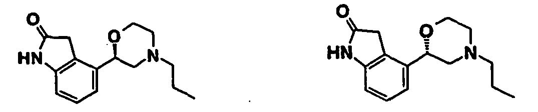

To a solution of the product of preparation 16 (880 mg, 2.1 mmol) in glacial acetic acid (20 mL) was added zinc dust (1.37 g; 21.1 mmol, 10 eq.) portionwise. The reaction mixture was stirred under nitrogen at r.t. for 1 h, then the acetic acid was removed *in vacuo* and the crude residue partitioned between EtOAc (100 mL) and sat. NaHCO₃ solution (50 mL). The organic layer was separated and the aqueous layer was extracted with EtOAc (2 x 100 mL). The combined organic layers were dried over Na₂SO₄ and evaporated to a brown oil. The crude material was purified by flash chromatography on SiO₂ twice eluting with DCM/MeOH/0.880 NH₃ 97/3/0.3 yielding 135 mg of racemic material as a beige solid (25% over 2 steps from the material of preparation 15).
M/S (APCI⁺) = 261 (MH⁺), (APCI⁻) = 259 (M⁻¹)
NMR (400MHz, CDCl₃) δ_{H}: 0.91 (t, 3H), 1.48-1.57 (m, 2H), 2.09 (t, 1H), 2.22 (td, 1H), 2.34 (t, 2H), 2.80 (d, 1H), 2.88 (d, 1 H), 3.60 (s, 2H), 3.82 (td, 1H), 4.02 (dd, 1 H), 4.58 (dd, 1H), 6.79 (d, 1 H), 7.02 (d, 1H), 7.18-7.22 (m, 1 H), 8.07 (brs, 1 H).

The racemate was split into its separate enantiomers using a Chiralpak AS-H column with a flow rate of 20 mL/min and eluting with methanol/ethanol 50/50.
Enantiomer 1:
   Retention time = 4.6min, ~100% e.e.
   M/S (APCI⁺) = 261 (MH⁺), (APCI⁻) = 259 (M⁻¹)
   NMR (400MHz, CDCl₃) - as above
   Elemental Analysis:
   C₁₅H₂₀N₂O₂.0.2H₂O requires C 68.3%, H 7.8%, N 10.6%. Found C 68.2%, H 7.6%, N 10.6%.
Enantiomer 2:
   Retention time = 6.5 min, ~100% e.e.
   M/S (APCl⁺) = 261 (MH⁺), (APCl⁻) = 259 (M⁻¹)
   NMR (400MHz, CDCl₃) - as above
   Elemental Analysis:
   C₁₅H₂₀N₂O₂.0.5H₂O requires C 66.9%, H 7.9%, N 10.4%. Found C 66.7%, H 7.7%. N 10.3%:

### Example 16

### 4-(1-propylpipendin-3-yl)-1H-indazole

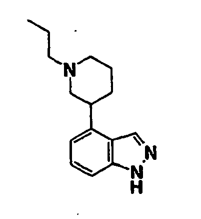

A solution of the product of preparation 19 (1.17 g, 3.21 mmol), in MeOH (20 mL) with PtO₂ (150 mg) was hydrogenated at r.t. and 60psi for 16 h. The catalyst was carefully filtered through a plug of arbocel® under a nitrogen atmosphere. The filtrate was evaporated to dryness and the residue purified by flash chromatography using DCM/MeOH 95/5 as eluent. This yielded 550 mg (71%) of *title compound* as beige foam.
M/S (APCl⁺) = 244 (MH⁺); (APCl⁻) = 242 (M-1)
NMR (400MHz, CDCl₃) δ _{H}: 0.97 (t, 3H); 1.91-2.02 (m, 3H); 2.11 (d, 1H); 2.24 (d, 1H); 2.50-2.65 (m, 1H); 2.75-2.85 (m, 2H); 2.90-3.00 (m, 2H); 3.73-3.83 (m, 2H); 4.16-4.27 (m, 1 H); 6.97 (d, 1 H); 7.32 (t, 1 H); 7.47 (d, 1H); 8.46 (s, 1 H); 10.66 (brs, 1 H).

### Example 17

### 4-[(2R,5S)-5-Methyl-4-propyl-morpholin-2-yl]-1,3-dihydro-indol-2-one

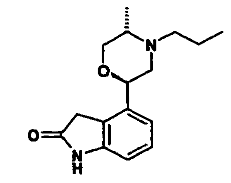

To a solution of 4-{1-Hydroxy-2-[((1*S*)-2-hydroxy-1-methyl-ethyl)-propyl-amino]-ethyl}-1,3-dihydro-indol-2-one (1.1 gms, 3.7mmol) in dry CH₂Cl₂ (9mls) split into 3 flasks was added c.H₂SO₄ (2mls per flask). The reaction mixture was stirred at room temperature for 45mins, then cooled to 0°C. Each reaction was then quenched with NH₄OH (aq) and contents combined and extracted with CH₂Cl₂ (4x75mls). The combined organic extracts were dried over MgSO₄, filtered and evaporated to dryness. The residue was chromatographed on silica eluting with CH₂Cl₂/MeOH/NH₃: 95/5/0.5 to 90/10/1. The relevant fractions were combined and evaporated to dryness. The residue remained impure so re-chromatographed on silica eluting with CH₂Cl₂/MeOH/NH₃: 9614/0.25. The relevant fractions were combined and evaporated to dryness to afford a beige gum (748mgs). This material was separated by HPLC on a Chiralpak AS-H column, isocratic elution with EtOH/MeOH (50:50) at a flow rate of 18mls/min to give desired diastereomer (187mgs, 36%, retention time 7.75mins) as beige solid.

TLC: CH₂Cl₂/MeOH/NH₃: 90/10/1 :R_{f}=0.46
M/S: APCI+ 275 (MH+)
¹H NMR:(400MHz, CDCl₃) δ ppm 0.89 (t, 3H), 1.05 (d, 3H), 1.50 (m, 2H), 2.29 (m, 2H), 2.46 (m, 1H), 2.75 (m, 1 H), 2.93 (dd, 1H), 3.42 (m, 1H), 3.59 (s, 2H), 3.86 (dd, 1H), 4.61 (d, 1H), 6.79 (d, 1H),7.03 (d, 1H), 7.20 (t, 1 H), 8.18 (bs, 1H)
Elemental Analysis: +0.10 H₂O Total MW=276.2

### Example 18

### N-[2-(1H-indazol-4-yl)ethyl]-N-propylamine

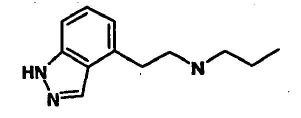

[2-(1-acetyl-1H-indazol-4-yl)-ethyl]-propyl-carbamic acid tert-butyl ester (2.5g, 7.23mmol) dissolved in 6N HCl _{(aq)} (25ml) and conc. HCl (25ml) added dropwise at 60C. The reaction was stirred at 60C for 1h. Dioxan (50ml) added, followed by cautious addition of ice. The mixture was extracted with dichloromethane (200ml). The aqueous layer was basified by cautious addition of 0.880 NH_{3 (aq)} (30ml) and extracted with dichloromethane (2x200ml) and then with EtOAc (3x200ml). The organic layers combined dried over MgSO₄ and solvent evaporated *in vacuo* to give 1.4g of a brown oil. The residue was dissolved in 91:9:1 EtOAc/MeOH/NH₄OH and flash chromatographed on SiO₂ column eluting with 91:9:1 EtOAc/MeOH/NH₄OH to give a pale yellow oil (800mg). This material was further purified by HPLC on a Phenomenex Gemini column with mobile phase 0.05% DEA in CH₃CN to give the product as a clear oil (540mg) ret.time 4.032min.
¹H NMR (400MHz, CD₃OD) δ(ppm):8.15 (s, 1H), 7.40 (d, 1H), 7.32 (t, 1H), 6.97 (d, 1H), 3.15 (t, 2H), 2.96 (t, 2H), 2.59 (t, 2H), 1.47-1.57 (m, 2H), 0.90 (t, 3H)
M/S APCl+ 204 (MH+)
M/S APCI- 202 (M-1)

### Examples 19 and 20

### 4-[(2R,5S)-5-methyl-4-ethylmorpholin-2-yl]-1H-indazole & 4-[(2S,5S)-5-methyl-4-ethylmorpholin-2-yl]-1H-indazole

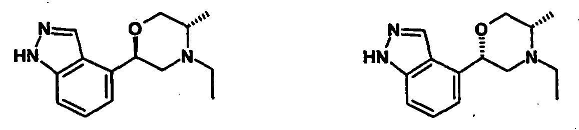

Concentrated H₂SO₄ (15ml) was added to (2*S*)-2-{[2-hydroxy-2-(2-trityl-2H-indazol-4-yl)-ethyl]-ethylamino}propan-1-ol (3.1g, 6.13mmol) in CH₂Cl₂ (100ml) at room temperature and stirred at room temperature for 1 hour. The reaction mixture was basified to pH9 by cautious addition of 0.880 NH₃, extracted with CH₂Cl₂ (3 x 100ml), and the organics combined, dried over MgSO₄, filtered and the solvent evaporated. This material was chromatographed on an lsco Combiflash Companion autochromatography system (120g SiO₂ column) eluting with a gradient from 100% CH₂Cl₂ to 60/40/4 CH₂Cl₂/MeOH/NH₄OH to give 1.0g of a mixture of diastereoisomers as a clear oil.

This mixture was separated by HPLC on a chiralpak AD-H column (250 x 21.5), at a flow rate of 18ml/minute with a mobile phase of 70:30 Hexane:IPA to give

Diastereoisomer 1, 190mg, retention time 5.859 min, white solid,
LRMS (APCI+) 246 (MH+)
LRMS (APCI-) 244 (M-1)
¹H NMR (400MHz, CD₃OD) δ(ppm): 1.02-1.13 (m, 6H), 2.36-2.48 (m, 2H), 2.55-2.67 (m, 1H), 2.94-3.11 (m, 2H), 3.49 (t, 1 H), 3.97 (m, 1H), 4.97 (m, 1H), 7.12 (d, 1H), 7.36 (t, 1 H), 7.47 (d, 1H), 8.19 (s, 1H)
Diastereoisomer 2, 360mg, retention time 14.519 min, white solid
LRMS (APCI+) 246 (MH+)
LRMS (APCI-) 244 (M-1)
¹H NMR (400MHz, CDCl₃) δ(ppm): 1.14 (t, 3H), 1.23 (d, 3H), 2.48-2.62 (m, 2H), 2.72-2.78 (m, 2H), 3.88-3.95 (m, 1H). 3.98-4.06 (m, 1H). 4.97 (m, 1H). 7.14 (d, 1H). 7.36 (t, 1H). 7.48 (d, 1H). 8.20 (s, 1H)

The following preparations illustrate the synthesis of certain intermediates used in the preparation of the preceding examples:

### Preparation 1

### 2-acetyl-4-bromo-2H-indazole

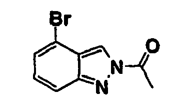

To 3-bromo-2-methylaniline (15.0 g, 81 mmol) in toluene (300 mL) under nitrogen at room temperature was added Ac₂O (23 mL, 240 mmol, 3 eq.) and KOAc (7.5 g, 8.32 mmol 1.02 eq). The resulting thick suspension was heated to 60°C for 30 minutes. Isoamylnitrite (16.4 mL, 121 mmol, 1.5 eq) was then added dropwise to this solution at 60°C and the reaction stirred at this temperature for 72 h. The reaction was allowed to cool, diluted with water (400 mL), extracted with EtOAc (500 mL). The organic extracts were dried over MgSO₄, filtered and evaporated *in vacuo* to give 18.8g of the *title compound* as an orange solid (98%)
¹H NMR (400MHz, CDCl₃) δ_{H} : 2.8(s, 3H), 7.4(t, 1H), 7.5(d, 1H), 8.15(s, 1H), 8.4(d, 1 H)
TLC 10% EtOAc in pentane Rf = 0.95

### Preparation 2

### 4-bromo-1H-indazole

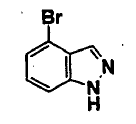

To 2-acetyl-4-bromo-2*H*-indazole (18.8 g, 79 mmol; preparation 1) in 5M aqueous HCl (100 mL) at 50°C was added concentrated hydrochloric acid (50 mL) dropwise over 15 minutes. The reaction was stirred at 60°C for 15 minutes. The reaction was allowed to cool and extracted with toluene (300 mL). The white solid that precipitated out was combined with the aqueous layer, basified to pH 10 and extracted with EtOAc (2 x 400 mL). The organic layers were combined, dried over MgSO₄, filtered and evaporated *in vacuo.* The crude material was dissolved in toluene and filtered through a pad of SiO₂, eluting with EtOAc, and solvent evaporated from the filtrate to yield 13.6 g (88%) of *title compound* as a solid.
M/S (ESI+) 197 (MH+)
¹H NMR (400MHz, CDCl₃) δ_{H} : 7.25(m, 1H), 7.35(d, 1H), 7.45(d, 1H), 8.15(s, 1H), 10.45-10.6 (brs, 1H) TLC 25% EtOAc in pentane Rf = 0.35

### Preparation 3

### 4-bromo-2-trityl-2H-indazole

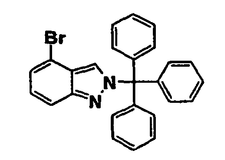

To 4-bromoindazole (1.3 g 6.6 mmol; preparation 2) in CH₂Cl₂ (10 mL) at r.t. was added triethylamine (1.84 mL, 13.2 mmol, 2 eq) and chlorotriphenylmethane (1.84 g, 6.6 mmol, 1 eq). The reaction was stirred at room temperature for 18 h, diluted with water (50 mL), extracted with CH₂Cl₂ (100 mL) and dried over MgSO₄. The solution was filtered, pre-absorbed onto SiO₂ and purified by flash chromatography on a SiO₂ column eluting with a gradient of EtOAc in pentane (2.5% to 10%) to yield 2g (69%) of a white solid.
¹H NMR (400MHz, CDCl₃) δ_{H} 7.1-7.4(m, 17H), 7.65(d, 1H), 7.9(s, 1H)
¹H NMR suggests a single regioisomer was obtained. The regiochemistry was tentatively assigned as shown above, and the trityl group described as being at this position throughout.

### Preparation 4

### (2S)-2-(propylamino)propan-1-ol hydrochloride

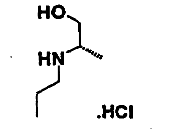

To (2*S*)-(+)-2-aminopropan-1-ol (19.6g, 0.26mol) dissolved in CH₂Cl₂ (500 mL) was added propionaldehyde (20.9 mL, 0.28 mol) followed by pre-dried, powdered 4Å molecular sieves (40g) and the mixture stirred at room temperature overnight. The mixture was filtered through a pad of celite® (filter agent), the pad washed with CH₂Cl₂, and solvent evaporated from the filtrate to give a clear oil. This oil was dissolved in methanol (200 mL) and NaBH₄ was added portionwise over 15 minutes. The mixture was stirred at r.t. overnight, then quenched by cautious addition of 2M aqueous HCl (200 mL), basified by addition of 2M aqueous NaOH (200 mL) and methanol removed by evaporation. Di-*ter*t*-*butyidicarbonate (115 g, 0.52 mol) was added to the residue followed by 1,4-dioxan (200 mL) and the mixture stirred at r.t. overnight. 1,4-dioxan was removed by evaporation and the residue diluted with water (750 mL) and extracted with CH₂Cl₂ (2 x 750 mL). The combined organic fractions were dried (MgSO₄), filtered and evaporated giving a clear oil. To this oil was added 4M HCI in 1,4-dioxan (200 mL) and the mixture stirred at r.t. overnight. The solvent was removed by evaporation to give the *title compound* as a white solid (24 g).
¹H NMR (DMSO, 400MHz) δ: 0.95 (t. 3H), 1.2 (d, 3H), 1.6 (m, 2H), 2.8 (m, 2H), 3.15 (m, 1H), 3.5 (bm, 1H). 3.6 (m, 1H). 5.4 (b, 1H). 8.6-8.9 (bd, 2H)
M/S (APCl⁺), 118 (MH⁺)

### Preparation 5

### (5S)-5-methyl-4-propylmorpholin-2-one

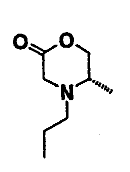

The material from preparation 4 (4 g, 26 mmol) was dissolved in benzene (80 mL), followed by the addition of *N*-ethyldiisopropylamine (9.07 mL, 52 mmol) and methyl bromoacetate (2.4 mL, 26 mmol). The mixture was heated to reflux with azeotropic removal of water overnight. After cooling to room temperature, the solvent was removed by evaporation, the crude material dissolved in methanol, pre-absorbed onto SiO₂ and purified by flash chromatography on SiO₂ eluting with 40% EtOAc in pentane to afford the *title compound* as a clear oil (1.78 g).
¹H NMR (CDCl₃, 400MHz) δ_{H} 0.9 (t, 3H), 1.1 (d, 3H), 1.5 (m, 2H), 2.25 (m, 1H), 2.6 (m, 1H), 2.8 (m, 1H), 3.2 (d, 1H), 3.6 (d, 1H), 4.05 (dd, 1H), 4.3 (dd, 1 H)
TLC. Rf=0.18 (50% EtOAc in pentane, UV visualisation)

### Preparation 6

### (5S)-5-methyl-4-propyl-2-(2-trityl-2H-indazol-4-yl)morpholin-2-ol

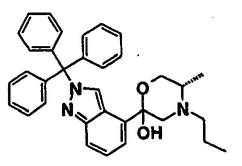

*t*-Butyllithium (1.7M in pentane, 6.7 mL, 11.4 mmol, 2 eq) was added dropwise to a stirring solution of 4-bromo-2-trityl-2H-indazole (2.53 g, 5.7 mmol, 1 eq; preparation 3) in THF (20 mL) maintaining a temperature of less than -70°C. (5*S*)-5-methyl-4-propylmorpholin-2-one (900 mg, 5.7 mmol, 1eq; preparation 5) was added immediately as a solution in THF (20 mL) and the reaction was allowed to proceed for 30 mins. The reaction was quenched by addition of NH₄Cl (60 ml, 10% w/v aqueous), allowed to warm to room temperature, and extracted with EtOAc (60 mL). The organic extracts were dried over MgSO₄, filtered, evaporated, and purified by flash chromatography on a SiO₂ column eluting with 50% EtOAc in pentane yielding 1.38g (47%) of *title compound* a pale yellow solid.
M/S (ESI⁺) 518 (MH⁺), 540 (MNa+)
M/S (ESI-) 516 (M-1)
TLC EtOAc, Rf=0.65

### Preparation 7

### (2S)-2-[[2-hydroxy-2-(2-trityl-2H-indazol-4-yl)ethyl](propyl)amino]propan-1-ol

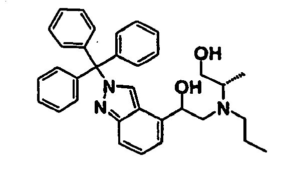

NaBH₄ (400 mg, 10.6 mmol, 4 eq) was added to a stirring solution of (5*S*)-5-methyl-4-propyl-2-(2-trityl-2*H-*indazol-4-yl)morpholin-2-ol (1.38 g, 2.6 mmol, 1eq; preparation 6) in EtOH (8 mL) and water (6 mL) at r.t, and the reaction was allowed to stir for 18 h. The reaction was quenched by addition of NH₄Cl (10% w/v aqueous) (50 mL), diluted with brine (50 mL) and extracted with EtOAc (200 mL). The organics were dried over MgSO₄, evaporated, and purified by flash chromatography on a SiO₂ eluting with 60% EtOAc in pentane yielding 660mg (48%) of *title compound* as a white solid.
M/S (ESI+) 520 (MH+), 542 (MNa+)
¹H NMR (400MHz, CDCl₃) δ_{H} : 0.8 (m, 3H), 0.9 (m, 3H), 1.2-1.6 (m, 2H), 2.35-3.1 (m, 5H), 3.2-3.4 (m, 2H), 4.8 (m, 1H), 7.0 (m, 1H), 7.15-7.4 (m, 16H), 7.65 (d, 1H), 8.05 (s, 1H)

### Preparation 8

### 4-propyl-2-(2-trityl-2H-indazol-4-yl)morpholin-2-ol

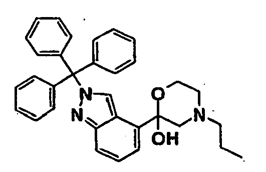

*t*-Butyllithium (1.7M in pentane, 8.0 mL, 13.6 mmol, 2 eq) was added dropwise to a stirring solution of 4-bromo-2-trityl-2*H*-indazole (3.0 g, 6.8 mmol, 1 eq; preparation 3) in THF (45 mL) maintaining a temperature of less than -70°C. 4-propylmorpholin-2-one (1.0 g, 7.0 mmol, 1.03 eq; preparation 17) in THF (5 mL) was added immediately and the reaction was allowed to proceed for 30 mins. The reaction was quenched by addition of brine (60 mL), allowed to warm to room temperature, and extracted with EtOAc (3 x 60 mL). The organic extracts were dried over MgSO₄, filtered, evaporated, and purified by flash chromatography on a SiO₂ eluting with a gradient from 98/2/0.2 to 97/3/0.3 CH₂Cl₂/MeOH/0.880 NH₃ yielding 1.70g (49%) of *title compound.*
M/S (APCI+) 504⁻(MH+)
¹H NMR (400MHz, CDCl₃) δ_{H} (compound exists as a mixture of ring open hydroxy ketone and ring closed lactol forms) : 0.8 (m, 3H), 1.4 (m, 2H), 1:9-2.2 (m, 4H), 2.6-2.8 (m, 1.5H), 3.4-3.6 (m, 1 H), 4.0 (m, 1 H), 4.5 (m, 0.5H), 6.2 (m, 0.5H), 6.8 (m, 0.25H), 7-7.6 (m, 17H), 7.95 (m, 0.25H), 8.1 (s, 0.75H), 8.45 (s, 0.25H)

### Preparation 9

### 2-[(2-hydroxyethyl)(propyl)amino]-1-(2-trityl-2H-indazol-4-yl)ethanol

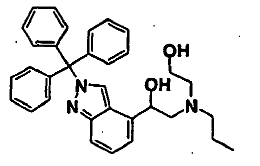

NaBH₄ (90 mg, 2.4 mmol, 4 eq) was added to a stirring solution of 4-propyl-2-(2-trityl-2*H*-indazol-4-yl)morpholin-2-ol (300 mg, 5.96 mmol, 1 eq; preparation 8) in EtOH (6 ml) and water (4 ml) at r.t., and the reaction was allowed to stir for 3h. The reaction was quenched by addition of brine (50 mL) and extracted with EtOAc (2 x 50mL). The organics were dried over MgSO₄, filtered and evaporated to give 305 mg (100%) of *title compound.*
M/S (APCI+) 506 (MH+)
¹H NMR (400MHz, CDCl₃) δ_{H} : 0.8-1.0 (t, 3H), 1.5-1.7 (m, 2H), 2.6-3.0 (m, 5H), 3.6-3.8 (m, 2H), 5.1-5.2 (m, 1H), 6.9-7.1 (m, 2H), 7.15-7.4 (m, 15H), 7.65 (d, 1 H), 8.05 (s, 1H)

### Preparation 10

### tert-butyl 3-(2-trityl-2H-indazol-4-yl)azetidine-1-carboxylate

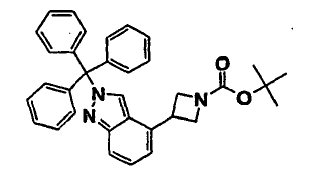

*tert*-Butyl 3-iodoazetidine-1-carboxylate (1 g, 3.5 mmol, 1 eq; prepared according to *Synlett,* 4, 1998, 379) was dissolved in dry DMF (12 mL), fresh Zn/Cu couple (400 mg) was added and the mixture sonicated at r.t. for 4 h. 4-bromo-2-trityl-2*H*-indazole (1.63 g, 3.7 mmol, 1.05 eq; preparation 3), tris(dibenzylideneacetone)dipalladium(O) (160 mg, 0.17 mmol, 0.05 eq) and tri-*o*-furylphosphine (85 mg, 0.35 mmol, 0.1 eq) were added and the mixture heated to 70°C for 18 h. After cooling to r.t., the mixture was diluted with saturated aqueous NH₄Cl (100 ml) and extracted with Et₂O (2 x 100mL). Organic extracts were combined, dried (MgSO₄), filtered and evaporated. The crude material was pre-absorbed onto SiO₂ and purified by flash chromatography on a SiO₂ column with a gradient elution from 7.5% - 60% EtOAc in pentane to give 600 mg (33%) of the *title compound* as a pale yellow solid.
M/S (ESI+) 516 (MH+), 538 (MNa+)
¹H NMR (400MHz, CDCl₃) δ_{H} 1.45 (s, 9H), 3.85-4.0 (m, 1H), 4.05-4.15 (m, 2H), 4.2-4.35 (m, 2H), 6.95 (d, 1H), 7.15-7.40 (m, 16H), 7.65 (d, 1H), 8.9 (s, 1 H)

### Preparation 11

### 4-azetidin-3-yl-1H-indazole trifluoroacetate

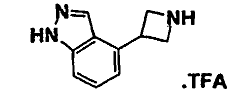

*tert*-Butyl 3-(2-trityl-2*H*-indazol-4-yl)azetidine-1-carboxylate (4.4 g, 8.5 mmol, 1eq; preparation 10), Et₃SiH (2.0 ml, 12.5 mmol, 1.5 eq), trifuoroacetic acid (20 ml) and CH₂Cl₂ (50 ml) were combined and stirred at r.t. for 90 min. Solvent removed *in vacuo* to give a mixture of product and triphenylmethane as a brown gum, containing an estimated 23 wt% of 4-azetidin-3-yl-1*H*-indazole trifluoroacetate. This material was carried forward crude to the reductive aminations of examples 6 to 12.

### Preparation 12

### 4-bromo-1-(triisopropytsilyl)-1H-indole

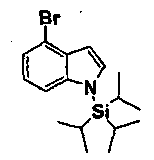

To a stirring suspension of NaH (3.35gms, 60% dispersion in oil, 84mmol) in dry THF (100mls) at 0°C was added 4-bromoindole (10mls, 80mmol) slowly. The reaction was stirred for 20mins then TIPSCI (17.8mls, 84mmol) was added and reaction stirred at room temperature over the weekend. The reaction was partitioned between H₂O (50mls) and EtOAc (50mls). The aqueous phase was re-extracted with EtOAc (2x100mls). The combined organic portions were dried over MgSO₄, filtered and evaporated to dryness. The residue was triturated with pentane then dried on high vacuum pump to yield product as beige solid (24.7gms, 88%)
TLC: EtOAc/Pentane 1:4 R_{f}=0.91
M/S: APCl+ 354 (MH+)
¹H NMR:(400MHz, CDCl₃) δ ppm 7.46 (d, 1H), 7.29 (m, 2H), 7.02 (m, 1H), 6.70 (d, 1H), 1.70 (m, 3H), 1.15 (d, 18H)

### Preparation 13

### 4-propyl-2-[1-(trisopropylsilyl)-1H-indol-4-yl]morpholin-2-ol

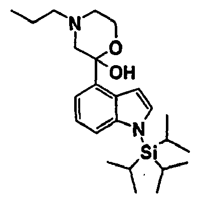

A dry 3-necked 500 mL round bottomed flask equipped with rubber septa, magnetic stirrer bar and nitrogen inlet and outlet was evacuated, back-filled with nitrogen several times, and charged with a solution of the compound of preparation 12 (6.6 g, 18.73 mmol) in dry THF (100 mL). The mixture was cooled to -78°C and *t*-BuLi (1.7M in pentane, 22ml, 37.5 mmol, 2.0 eq.) was added dropwise. The mixture was stirred at -78°C for 5 minutes before the addition of a solution of 4-propylmorpholin-2-one (2.68 g, 18.73 mmol, 1.0eq.; preparation 17) in THF (15 mL) dropwise. The reaction mixture was stirred at -78°C for 45 minutes then quenched with 20 mL sat. NH₄Cl solution and allowed to warm to r.t. overnight. The reaction mixture was partitioned between EtOAc (100 mL) and water (50mL) and the aqueous layer extracted with EtOAc (2 x 100 mL). The combined organics were dried over Na₂SO₄ and evaporated to a yellow oil which was purified by flash chromatography on SiO₂ using a gradient eluent of pentane/EtOAc 4/1 to 2/1 to 1/1 to 0/100 and the fractions containing product combined and evaporated yielding the *title compound* as a pale yellow oil (4.3 g, 68% yield).
TLC Pentane/EtOAc 4:1 Rf = 0.2

### Preparation 14

### 2-[(2-hydroxyethyl)(propyl)amino]-1-[1-(triisopropylsilyl)-1H-indol-4-yl]ethanol

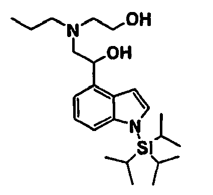

To a solution of the product of preparation 13 (4.3 g, 10.32 mmol) in a mixture of EtOH (25 mL) and water (15 mL) was added NaBH₄ (1.56 g, 41.3 mmol, 4.0 eq.) portionwise over 5 minutes. After 20 minutes the reaction was judged complete by TLC. The reaction mixture was quenched with 30 mL sat. NH₄Cl solution and extracted with EtOAc (3 x 50 mL). The combined organics were dried over Na₂SO₄ and evaporated to give 3.8g of a yellow oil. This was used directly in the next step without further purification.

### Preparation 15

### 4-(4-propylmorpholin-2-yl)-1H-indole

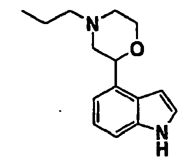

To a solution of the product of preparation 14 (3.8 g) in dry DCM (25 mL) at 3°C under nitrogen was added 2 mL of concenrated H₂SO₄ dropwise. The 2 phase reaction mixture was stirred vigorously and allowed to warm to r.t. over 1 h. Another 10ml of concentrated H₂SO₄ was then added and the reaction mixture stirred for another 2h until judged complete by TLC. The reaction mixture was carefully poured onto ice and basified with 0.880 NH₃ solution (100 mL). The mixture was then extracted with EtOAc (3 x 150 mL) and the combined organics were dried over Na₂SO₄ and evaporated to dryness. The crude material was purified by chromatography on SiO₂ once using DCM/MeOH/0.880 NH₃ 99/1/0.1 as eluent, then again using DCM/MeOH/0.880 NH₃ 100/0/0 to 99/1/0.1 yielding the *title compound* as a beige oil (630mg, 28% over 2 steps).
M/S (APCI⁺) = 245 (MH⁺)
NMR (400MHz, CDCl₃) δ_{H} : 0.91(t, 3H), 1.49-1.59(m, 2H), 2.22-2.37(m, 4H), 2.86(d, 1H), 3.08(d, 1H), 3.94(td, 1H), 4.10(dd, 1H), 5.00(dd, 1H), 6.69(s, 1H), 7.18(d, 1H), 7.19-7.22(m, 1H), 7.30-7.34(m, 1H), 8.21(bs, 1H).

### Preparation 16

### 3,3-dibromo-4-(4-propylmorpholin-2-yl)-1,3-dihydro-2H-indol-2-one

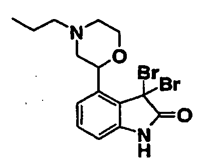

To a solution of the product of preparation 15 (515 mg, 2.11 mmol) in *t*-BuOH (20 mL) at r.t. under nitrogen was added pyridinium tribromide (2.02 g, 6.32 mmol, 3.0 eq.) portionwise over 15 minutes. The reaction mixture was stirred for 20 h at r.t. The mixture was partitioned between EtOAc (100 mL) and sat. NaHCO₃ solution (50 mL) and the aqueous layer was extracted with EtOAc (2 x 100 mL). The combined organics were dried over Na₂SO₄ and evaporated to a brown solid. This was used directly in the next step without further purification.

### Preparation 17

### 4-propylmorpholine-2-one

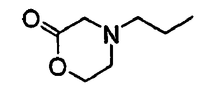

Methyl 2-bromoacetate (50 mL, 0.54 mol, 1 eq) was added slowly to N-propylaminoethanol (62.4 ml, 0.54 mol, 1 eq) and Et₃N (75 ml, 0.54 mol, 1eq) in toluene at 0°C and allowed to stir at room temperature overnight. Water (1 L) was added, and the mixture extracted with EtOAc (2 x 500 mL). Brine (500 mL) was added to the aqueous layer, which was re-extracted with EtOAc (2 x 500mL). Organic layers were combined, dried (MgSO₄), filtered and solvent removed *in vacuo* to give 62.7g (81%) of *title compound* as a clear oil.
TLC EtOAc Rf =0.5
M/S (APCI+) 144 (MH+)
¹H NMR (400Mhz, CD₃OD) δ_{H} 0.9 (t, 3H), 1.4-1.6 (m, 2H), 2.3-2.4 (m, 2H), 2.6-2.7 (m, 2H), 3.3 (s, 2H), 4.4 (m, 2H)

### Preparation 18

### 4-pyridin-3-yl-2-trityl-2H-indazole

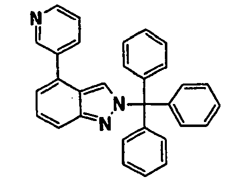

To a solution of the product of preparation 3 (4.39 g, 10.0 mmol) in toluene (80 mL) and ethanol (50 mL) was added diethyl-(3-pyridyl)-borane (commercially available, 1.47 g, 10.0 mmol), tetrakis(triphenylphosphine)palladium(0) (577 mg, 0.50 mmol) and sodium carbonate (1.59g, 15.0mmol as a solution in water 2 mL). The reaction mixture was refluxed for 4 h, then cooled, diluted with ethyl acetate (100 mL) and washed with water (100 mL). The organic layer was separated and the aqueous layer was extracted with EtOAc (2 x 80mL). The combined organic layers were dried over Na₂SO₄ and evaporated to a yellow oil. This was taken up in pentane/EtOAc 1:1 (20 mL) which induced crystallisation. The solvent was evaporated and the solid triturated with hot ethanol and the solid collected on a filter yielding the *title compound* (2.32 g, 53%) as a cream solid.
MS (APCl⁺) = 438 (MH⁺)
NMR (400MHz, CDCl₃) δ_{H}: 7.12-7.40 (m, 7H); 7.27-7.39 (m, 11H); 7.79 (d, 1H); 7.85 (dd, 1H); 8.04 (s, 1 H); 8.57 (d, 1 H); 8.84 (s, 1H)

### Preparation 19

### 3-(1H-indazol-4-yl)-1-propylpyridinium iodide

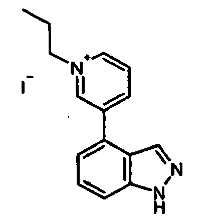

To a solution of the product of preparation 18 (2.32 g, 5.30 mmol) in acetonitrile (40 mL) was added propyl iodide (2.6 mL, 26.5 mmol) and the reaction mixture refluxed under nitrogen for 16 h. The mixture was evaporated to dryness and the crude material triturated with DCM/MeOH 95/5. The solid was collected by filteration and washed well with DCM/MeOH 95/5. This solid was dissolved in MeOH and filtered to remove a dark insoluble impurity. The filtrate was evaporated to yield the *title compound* as a brown solid (1.17 g, 61%).
NMR (400 MHz, CD₃OD) δ_{H}: 1.08 (t, 3H); 2.10-2.20 (m, 2H); 4.76 (t, 3H); 7.52 (d, 1H); 7.60 (t, 1H); 7.77 (d, 1H); 8.26 (t, 1H); 8.32 (s, 1H); 8.95 (d, 1H); 9.08 (d, 1 H); 9.42 (s, 1H).

### Preparation 20

### (5S)-5-Methyl-4-propyl-2-(1-triisopropylsilanyl-1H_indol-4-yl)-morpholin-2-ol

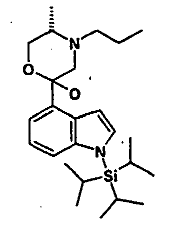

To a solution of 4-bromo-1-triisopropylsilanyl-1H-indole (6.0gms, 17mmol) in dry THF (85mls) at -78 °C was added *tert*-butyl lithium (20mls, 1.7M in hexanes, 34mmol) dropwise and the resultant solution was stirred for 10 mins. To this was added solution of 5-methyl-4-propyl-morpholin-2-one (2.68gms, 17mmol) in dry THF (15mls). The reaction was stirred at -78 °C for 1hr then at room temperature overnight. The reaction was partitioned between H₂O (50mls) and EtOAc (50mls) and organic phase extracted. The aqueous phase was re-extracted with EtOAc (3x50mls). The combined organic portions were dried over MgSO₄, filtered and evaporated to dryness. The residue was chromatographed on silica eluting with EtOAc/Pentane (1:3 to 1:1 to 1:0) to yield desired product (4.36gms, 59%)
TLC: EtOAc/Pentane 1:1 R_{f}=0.32
M/S: APCI+ 431 (MH+)
¹H NMR:(400MHz, CDCl₃) product is a mixture of ring opened ketone and ring closed lactol and NMR spectrum is impossible to assign without further NMR experiments being undertaken.

### Preparation 21

### (2S)-2-{[(2-Hydroxy-2-(1-triisopropylsitanyl-1H-indol-4-yl)-ethyl]-propyl-amino}-propan-1ol

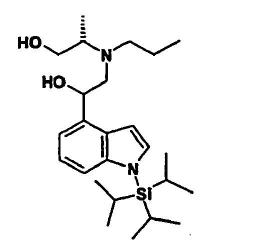

To a solution of (5*S*)-5-methyl-4-propyl-2-(1-triisopropylsilanyl-1H_indol-4-yl)-morpholin-2-ol (4.35gms, 10.1mmol) in EtOH (25mls) and H₂O (15mls) was added NaBH₄ (1.53gms, 40.5mmol) and the reaction mixture stirred for 1 hr. The reaction mixture was diluted with saturated NH₄Cl (aq) (10mls) and extracted with CH₂Cl₂ (3x20mls). The combined organic extracts were dried over MgSO₄, filtered and evaporated to dryness to yield title product as a mixture of diastereoisomers (4.16gms, 95%).
TLC: CH₂Cl₂/MeOH/NH₃: 93/7/1 :R_{f}=0.91
M/S: APCI+ 433 (MH+)
¹H NMR:(400MHz, CDCl₃) δ ppm 7.44 (d, 1H), 7.26 (m, 1H), 7.09-7.20 (m, 2H), 6.72 (dd, 1H), 5.09 (m, 1H), 3.43-3.53 (m, 1H), 3.26-3.36 (m, 1 H), 2.98-3.13 (m, 2H), 2.83 (m, 1H), 2.40-2.68 (m, 2H), 1.70 (m, 3H), 1.48-1.65 (m, 1 H), 1.29-1.48 (m, 1H), 1.13 (d, 18H), 0.96 (t, 1.5H), 0.92 (d, 1.5H), 0.85 (d, 1.5H), 0.79 (t, 1.5H).

### Preparation 22

### (2S)-2-{[2-(3-Chloro-1-triisopropylsilanyl_1H-indol-4-yl)-2-hydroxy-ethyl]-propyl-amino}-propan-1-ol

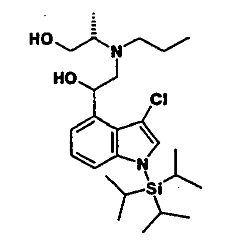

To a stirring solution of (2*S*)-2-{[(2-hydroxy-2-(1-triisopropylsilanyl-1H-indo-yl)-ethyl]-propyl-amino}-propan-1-ol (3.72gms, 8.60mmol) in AcOH (30mls) at room temperature was added N-chlorosuccinimide (1.38gms, 10.3mmol) in 3 portions. The reaction was stirred for 75mins before it was quenched with 2N NaOH (aq) and extracted with EtOAc (4x50mls). The combined organic extracts were dried over MgSO₄, filtered and evaporated to dryness. The residue was chromatographed on silica eluting with CH₂Cl₂/MeOH/NH₃: 97/3/0.25 to 9317/1, to yield desired compound as a mixture of diastereoisomers as a brown oil. (3.24gms, 80%)
TLC: CH₂Cl₂/MeOH/NH₃: 93/7/1 :Rᵣ=0.40
M/S: APCl+ 467 (MH+)
¹H NMR:(400MHz, CDCl₃) δ ppm 0.87 (t, 3H), 0.96 (d, 3H), 1.13 (d, 18H), 1.52 (m, 2H), 1.65 (m, 3H), 2.54 (m, 2H), 2.65 (m, 2H), 2.94 (dd, ½H), 3.17 (dd, %H), 3.48 (m, 2H), 5.86 (m, 1 H), 7.19 (m, 2H), 7.38 (d, 1 H), 7.43 (d, ½H), 7.48 (d, ½H)

### Preparation 23

### 4-{1-Hydroxy-2-[((1S)-2-hydroxy-1-methyl-ethyl)-propyl-amino]-ethyl}-1,3-dihydro-indol-2-one

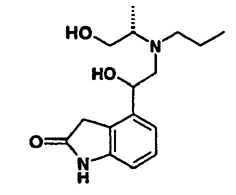

(2*S*)-2-{[2-(3-Chloro-1-triisopropylsilanyl_1H-indol-4-yl)-2-hydroxy-ethyl]-propyl-amino}-propan-1-ol (148mgs, 0.31mmol) was stirred in 2N HCl (aq) (10mls) for 2 hrs. The reaction mixture was basified with 2N NaOH (aq) and extracted with EtOAc (3x30mls). The combined organic extracts were dried over MgSO₄, filtered and evaporated to dryness. The residue was chromatographed on silica eluting with CH₂Cl₂/MeOH/NH₃: 96/4/0.5, to yield desired compound as reddish oil. (44mgs, 48%)
TLC: CH₂Cl₂/MeOH/NH₃: 90/10/1 :R_{f}=0.38
M/S: APCl+ 293 (MH+)
¹H NMR:(400MHz, CDCl₃) δ ppm 0.89 (t, 3H), 0.97 (d, 3H), 1.53 (m, 2H), 2.45-2.64 (m, 3½H), 2.84 (dd, ½H). 3.07 (m, 1H), 3.45-3.65 (m, 4H), 4.70 (m, 1H), 6.78 (d, 1H), 7.04 (d, 1H), 7.20 (t, 1H), 8.11 (bs, 1H)

### Preparation 24

### 2-methyl-3-nitrobenzyl methanesulfonate

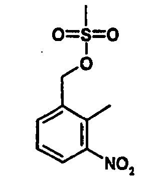

To (2-methyl-3-nitrophenyl)methanol (1g, 5.9mmol) and Et₃N (1.7ml, 11.9mmol, 2eq) in dichloromethane (5ml) at -10C was added MeSO₂Cl (560ul, 7.1mmol, 1.2eq) in dichloromethane (5ml) dropwise over 5min. The reaction was allowed to warm to r.t. and stirred at r.t. for 18h. The reaction was quenched by cautious addition of water (100ml), extracted with dichloromethane (100ml), organics separated, dried over MgSO₄ and solvent evaporated to give the product as an orange oil (1g 68%). Material carried forward with no further purification.

### Preparation 25

### (2-methyl-3-nitrophenyl)acetonitrile

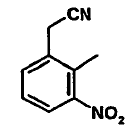

To 2-methyl-3-nitrobenzyl methanesulfonate (1g, 4mmol) in DMF (10ml) was added KCN (265mg, 4mmol, 1eq) and stirred at r.t. for 72h. The solvent was evaporated *in vacuo* and the residue partitioned between 2N NaOH (100ml) and Et₂O (100ml). The organics were separated, dried over MgSO₄ and the solvent was evaporated *in vacuo.* The crude material was dissolved in toluene and flash chromatographed on SiO₂ column, eluting with 15% EtOAc/Pentane to yield 530mg (73%) of a white solid.

¹H NMR (400MHz, CDCl₃) δ(ppm) : 7.78(d, 1H), 7.65(d, 1H), 7.40(t, 1H), 3.78(s, 2H), 2.46 (s, 3H)

### Preparation 26

### (2-methyl-3-nitrophenyl)acetic acid

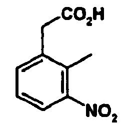

To (2-methyl-3-nitrophenyl)acetonitrile (500mg 2.8mmol) in ethanol (30ml) was added 20% w/v KOH_{(aq)} (30ml) and the mixture heated to reflux for 18h. After cooling, the mixture was extracted with dichloromethane (50ml) the aqueous layer separated, acidified to pH2 with 2N HCl_{(aq)} and extracted with EtOAc (50ml) and dichloromethane (50ml). The organic extracts were combined, dried over MgSO₄ and solvent evaporated *in vacuo* to give the product as a light brown solid (470mg, 86%).

¹H NMR (400MHz, CD₃OD) δ(ppm): 7.66(d, 1H), 7.50(d, 1H), 7.34(t, 1H), 3.79 (s,2H), 2.37(s, 3H)

### Preparation 27

### 2-(2-methyl-3-nitrophenyl)-N-propylacetamide

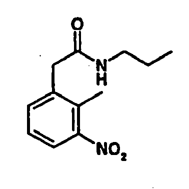

(2-methyl-3-nitrophenyl)acetic acid (450mg, 2.3mmol), propylamine (230uL, 2.7mmol, 1.2eq), 1-(3-dimethylaminopropyl)-3-ethylcarbodiimide (530mg, 2.7mmol, 1.2eq) 1-hydroxybenzotriazole (375mg, 2.7mmol, 1.2eq) and triethylamine (640uL, 4.6mmol, 2eq) were combined in DMF (10ml) and stirred at r.t. for 18h. The solvent was removed *in vacuo,* and the residue partitioned between water (50ml) and EtOAc (2x50ml). The organic layers were combined, dried over MgSO₄ and solvent evaporated *in vacuo* to give the product as a pale orange solid (430mg, 79%).

¹H NMR (400MHz, CD₃OD) δ(ppm): 7.65(d, 1H), 7.48(d, 1H), 7.34(t, 1H), 3.79 (s,2H), 2.37(s, 3H), 1.54 (m, 2H), 0.93 (t, 3H)
M/S (APCI+) 237 (MH+)
M/S (APCI-) 235 (M-1)

### Preparation 28

### N-[2-(2-methyl-3-nitrophenyl)ethyl]-N-propylamine

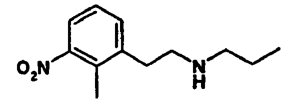

BH₃.THF (5.5ml, 5.5mmol, 1.0M in THF, 3eq) was added at r.t. to 2-(2-methyl-3-nitrophenyl)-N-propylacetamide (430mg, 1.8mmol) in THF (5ml) and the mixture heated to reflux for 4h. The reaction was allowed to cool to r.t., quenched with MeOH and the solvent removed *in vacuo*. 6M HCl_{(aq)} was added and the mixture heated to reflux for 2h. The mixture was allowed to cool, diluted with water (20ml) extracted with EtOAc (50mL) and the layers separated. The aqueous layer was basified by cautious addition of 0.880 NH₃ (aq) to pH10 and extracted with EtOAc (50mL) and dichloromethane (50mL). The organic layers were combined, dried over MgSO₄ and solvent evaporated *in vacuo* to give the product as a light brown oil (180mg, 45%)

¹H NMR (400MHz, CDCl₃) δ(ppm): 7.60 (d, 1H), 7.39 (d, 1H), 7.23 (t, 1H), 2.80 - 2.95 (m, 4H), 2.61 (t, 2H), 2.43 (s, 3H), 1.48-1.56 (m, 2H), 0.93 (t, 3H)
M/S (APCI+) 223 MH+

### Preparation 29

### [2-(2-Methyl-3-nitrophenyl)-ethyl]-propylcarbamic acid tert-butyl ester

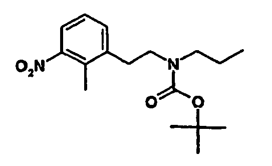

*N*-[2-(2-methyl-3-nitrophenyl)ethyl]-*N*-propylamine (180mg, 0.8mmol) and di-tert-butyl-dicarbonate (180mg, 0.8mmol 1eq) combined in dichloromethane (10ml) and stirred at r.t. for 4 h. Diluted with water (50ml) and extracted with dichloromethane (2x50ml), the organic layers combined dried over MgSO₄ and solvent evaporated *in vacuo.* The residue was dissolved in toluene and flash chromatographed on SiO₂ column eluting with 7% EtOAc/Pentane to give the product as a clear oil (160mg, 61%).
¹H NMR (400MHz, CD₃OD) δ(ppm): 7.60 (d, 1H), 7.35-7.48 (br m, 1H), 7.30 (t, 3H), 3.35-3.48 (br m 2H), 3.07-3.22 (br m, 2H), 2.98 (t, 2H), 2.42 (s, 3H), 1.25-1.62 (br m, 11H), 0.87 (br t, 3H)
M/S (APCl+) 223 (M-Boc H+)

### Preparation 30

### [2-(3-amino-2-methylphenyl)-ethyl]-propylcarbamic acid tert-butyl ester

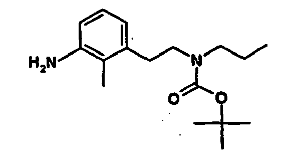

[2-(2-Methyl-3-nitrophenyl)-ethyl]-propylcarbamic acid tert-butyl ester (160mg, 0.5mmol), iron powder (70mg, 1.24mmol, 2.5eq) and NH₄Cl (30mg, 0.52mmol 1.05eq) combined in water (1ml) and EtOH (3ml) and heated to 80C for 18h. Filtered through Celite pad, washing with EtOH (50ml) and solvent removed *in vacuo.* The residue was dissolved in Et₂O and washed with 10% w/v K₂CO₃ (aq) (50ml). The organics were dried over MgSO₄ and solvent evaporated *in vacuo* to give the product as a light brown oil (125mg, 87%).
¹H NMR (400MHz, CD₃OD) δ(ppm): 6.86 (t, 1H), 6.62 (d, 1H), 6.50-6.59 (br m, 1H), 3.28-3.37 (br m, 2H), 3.0-3.15 (br m, 2H), 2.81 (t, 2H), 2.13 (s, 3H), 1.37-1.40 (m, 11 H), 0.81-0.88 (br m, 3H) M/S APCI+ 193 (M-Boc H+)

### Preparation 31

### [2-(1-acetyl-1H-indazol-4-yl)-ethyl]-propyl-carbamic acid tert-butyl ester

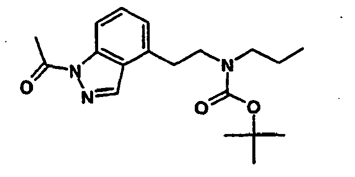

KOAc (40mg, 0.45mmol, 1.05eq) and acetic anhydride (120uL, 1.3mmol, 3eq) added to [2-(3-amino-2-methylphenyl)-ethyl]-propylcarbamic acid tert-butyl ester (125mg, 0.43mmol 1eq) in toluene (5ml) and the mixture heated to 60C for 30min. lsoamyl nitrite (90uL, 0.64mmol, 1.5eq) added dropwise and the reaction heated to 60C for 18h. The mixture was allowed to cool to r.t. and diluted with 2N NaOH _{(aq)} and water (50ml) and extracted with EtOAc (50ml). Layers seprated, and organics were dried over MgSO₄ and solvent evaporated *in vacuo* to give the product as an orange oil (130mg, 88%). This material was carried forward to deprotection without further purification.
M/S APCI- 302 (M-acetate-1)

### Preparation 32

### (S)-2-ethylamino-propan-1-ol

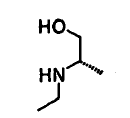

To (S)-(+)-2-amino-1-propanol (5g, 66.5 mmol) dissolved in dichloromethane (100ml) was added acetaldehyde (4.2ml, 73.2 mmol) followed by pre-dried powdered 4A molecular sieves (10g) and the mixture stirred at room temperature overnight. The mixture was filtered through a pad of celite, the pad washed with dichloromethane, and solvent evaporated to give a clear oil. This oil was dissolved in ethanol (60ml) and hydrogenated over Pt₂O catalyst (600mg) at a pressure of 30psi H₂ overnight. The reaction mixture was filtered through a pad of Arbocel, and the solvent was removed by evaporation to give a light brown oil (6.2g).
¹H NMR (CD₃OD, 400MHz) δ: 1.02 (d, 3H), 1.14 (t, 3H), 2.56-2.62 (m, 1H), 2.66-2.79 (m, 2H), 3.37-3.52 (m, 2H)
LRMS (APCI+), 104 (MH⁺)

### Preparation 33

### (5S)-5-methyl-4-ethylmorpholin-2-one

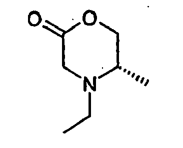

(S)-2-ethylamino-propan-1-ol (5.7g, 55.2 mmol) was dissolved in toluene (60ml) followed by the addition of N-ethyldiisopropylamine (9.6ml, 55.2 mmol) and methyl bromoacetate (5.0ml, 55.2 mmol). The mixture was heated to 50°C overnight. The mixture was allowed to cool to room temperature, diluted with water (100ml) and extracted with CH₂Cl₂ (3x100ml), dried over MgSO₄, filtered and solvent was removed by evaporation to give a light brown oil (4.9g). This material was dissolved in CH₂Cl₂ and chromatographed on an lsco Combiflash Companion autochromatography system (120g SiO₂ column) with a gradient elution from 100% CH₂Cl₂ to 90:10:1 CH₂Cl₂:MeOH:NH₄OH. Solvent containing fractions were evaporated to give a golden brown oil (2.9g)
¹H NMR (CDCl₃, 400MHz) δ: 1.00-1.10 (m, 6H), 2.33-2.42 (m, 1H), 2.68-2.82 (m, 2H), 3.15-3.60 (m, 2H), 3.97-4.06 (m, 1H), 4.23-4.31 (m, 1 H)
LRMS (APCI+), 144 (MH⁺)

### Preparation 34

### (5S)-5-Methyl-4-ethyl-2-(2-trityl-2H-indazol-4-yl)-morpholin-2-ol

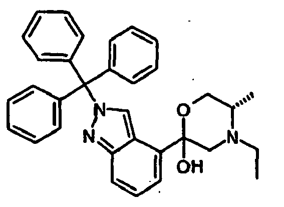

t-butyllithium (1.7M in pentane, 22.8ml, 38.7mmol, 2eq) was added dropwise to a stirring solution of 4-bromo-2-trityl-2H-indazole (8.5g, 19.3 mmol, 1eq; preparation 3) in THF (65ml) maintaining a temperature of <-70°C. (5*S*)-5-methyl-4-ethylmorpholin-2-one (2.8g, 19.3 mmol, 1eq) in THF (20ml) was added immediately and the reaction was allowed to proceed for 30 mins. The reaction was quenched by addition of water (200 ml), allowed to warm to room temperature, and extracted with EtOAc (2x200ml). The organic extracts were dried over MgSO₄, filtered, evaporated, and flash chromatographed through SiO₂ eluting with a gradient from 50% EtOAc/Pentane to 100% EtOAc yielding 4.0g of a pale yellow solid as a mixture of two diastereoisomers.
M/S (APCI-) 260 (M-1)

### Preparation 35

### (2S)-2-{[2-hydroxy-2-(2-trityl-2H-indazol-4-yl)-ethyl]-ethyl-amino}propan-1-ol

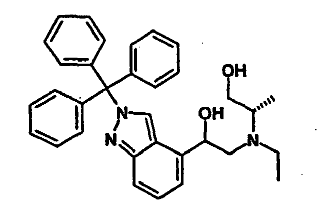

NaBH₄ (1.2g, 31.8mmol, 4eq) was added to a stirring solution of (5S)-5-Methyl-4-ethyl-2-(2-trityl-2*H-*indazol-4-yl)-morpholin-2-ol (4.0g, 7.9mmol, 1eq) in EtOH (80ml) and water (20ml) at room temperature, and the reaction was allowed to stir at room temperature for 18h. The reaction was quenched by addition of NH₄Cl (10% w/v aq) (100ml) and extracted with EtOAc (2x200ml). The organics were dried over MgSO₄, filtered, evaporated, and flash chromatographed on an lsco Combiflash Companion autochromatography system (120g SiO₂ column) eluting with a gradient from 100% EtOAc to 5%MeOH/EtOAc yielding 3.5g of a white solid as a mixture of two diastereoisomers.
M/S (APCl+) 507 (MH+)
¹H NMR (400MHz, CD₃OD) δ: (ppm) : 0.8 (d, 1.5H), 0.83-0.93 (m, 3.0H), 1.01 (t, 1.5H), 2.46-2.99 (m, 5H) , 3.18 (d, 1H), 3.30 (d, 1H) 4.77-4.83 (m. 1H), 7.04 (d, 1H), 7.10-7.19 (m, 6H), 7.22-7.28 (m, 1H), 7.31-7.40 (m, 9H), 7.50 (d, 1H), 8.19 (m, 1H)

## Claims

1. A compound of formula (I) 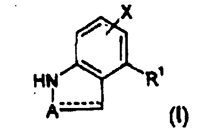 wherein:
A is selected from N or C=O
X is selected from H, methyl, ethyl, OH, OCH₃, OCH₂CH₃, halo, SCH₃, CN or CF₃
----- (the dashed bond in formula (I)) represents a single bond when A is N, and is absent when A is C=O
R¹ is selected from: 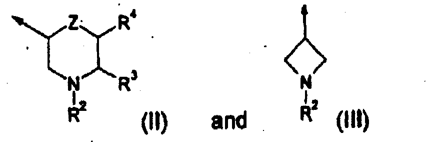
wherein:
Z represents O or CH₂;
R² represents H or (C₁-C₆)alkyl; wherein (C₁-C₆)alkyl may be optionally substituted by (C₁-C₆)alkyl, OR⁸, phenyl, or heteroaryl;
R³ represents H or (C₁-C₆)alkyl; wherein (C₁-C₆)alkyl may be optionally substituted by OR⁶;
R⁴ represents H or (C₁-C₆)alkyl;
R⁸ represents H, (C₁-C₆)alkyl, phenyl, or (CH₂)phenyl;
wherein heteroaryl means a 5 to 7 membered aromatic ring, containing from 1 to 4 heteroatoms, said heteroatom independently selected from O, S and N; said 5 heteroaryl may be optionally substituted with 1 or more substituents selected from (C₁-C₆)alkyl, halo and OR⁵, each substituent may be the same or different; and
wherein phenyl may be optionally substituted with 1 or more substituents selected from (C₁-C₆)alkyl, halo and OR⁸, each substituent may be the same or different, or a pharmaceutically acceptable salt, solvate or polymorph thereof, with the proviso that: Z is O when A is C=O.

2. A compound of formula (I) 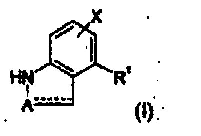 wherein:
A is selected from N or C=O
X is H
----- (the dashed bond in formula (l)) represents a single bond when A is N, and is absent when A is C=O
R¹ is 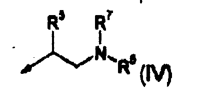
wherein:
R⁵ represents H, methyl, ethyl, methoxy, or ethoxy;
R⁸ represents (C₁-C₆)alkyl;
R⁷ represents H or (C₁-C₈)alkyl; wherein said (C₁-C₈)alkyl may be optionally substituted with 1 or 2 substituents each independently selected from OR⁸, phenyl or substituted phenyl;
R⁸ is as defined in claim 1;
wherein phenyl is as defined in claim 1;
or a pharmaceutically acceptable salt, solvate or polymorph
thereof, with the proviso that:
when A is C=O, then R⁵ cannot be H.

3. A compound according to claim 1 wherein X is selected from H, OH, F or CN.

4. A compound according to claim 1 or claim 3 wherein R¹ is moiety (II).

5. A compound according to claim 4 wherein Z is O; R² is (C₁-C₆)alkyl; R³ is selected from H or (C₁-C₆)alkyl; and R⁴ is H.

6. A compound according to claim 1 or claim 3 wherein R¹ is moiety (III).

7. A compound according to claim 6 wherein R² is (C₁-C₄)alkyl, optionally substituted by phenyl or heteroaryl.

8. A compound according to claim 2 wherein R⁵ is selected from H and methoxy; and R⁷ is selected from H and methyl.

9. A compound selected from:
4-[(2*R*-5*S*)-5-methyl-4-propylmorpholin-2-yl]-1*H*-indazole;
4-[1-(2-ethyl)azetidin-3-yl]-1*H*-indazole;
4-[1-(3-phenylpropyl)azetidin-3-yl])-1*H*-indazole;
4-(1-propylazetidin-3-yl)-1 *H*-indazole;
4-[(2*R*)4-propylmopholin-2-yl]-1,3-dihydro-2*H*-indol-2-one,
4-[(5*S*)-5-methyl-4-propyl-morpholin-2-yl]-1,3-dihydro-indol-2-one;
*N*-[2-(1*H*-indazol-4-yl)ethyl]-*N*-propylamine; and
4-[(5S)-4-ethyl-5-methylmorpholin-2-yl]-1H-indazole.

10. A compound according to any of claims 1 to 9 for use as a pharmaceutical.

11. The use of a compound according to any of claims 1 to 9 in the preparation of a medicament for the treatment of sexual dysfunction.

12. The use according to claim 11 wherein the sexual dysfunction is male erectile dysfunction or female sexual dysfunction.

13. The use of a compound according to any of claims 1 to 9 in the preparation of a medicament for the treatment of depression or psychiatric disorders.

14. The use of a compound according to any of claims 1 to 9 in the preparation of a medicament for the treatment of neurodegeneration.

15. A pharmaceutical composition comprising a compound according to any of claims 1 to 9, and a pharmaceutically acceptable diluent or carrier.

## Patentansprüche

1. Verbindung der Formel (I) 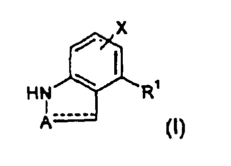 worin:
A aus N und C=O ausgewählt ist,
X ausgewählt ist aus H, Methyl, Ethyl, OH, OCH₃, OCH₂CH₃, Halogen, SCH₃, CN und CF₃,
----- (die gestrichelte Bindung in Formel (I)) eine Einfachbindung darstellt, wenn A N ist, und nicht vorhanden ist, wenn A C=O ist,
R¹ ausgewählt ist aus: 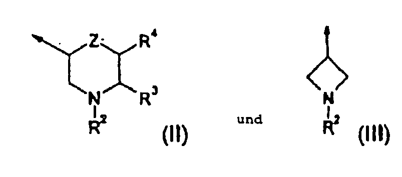
worin:
Z O oder CH₂ darstellt;
R² H oder (C₁-C₆)Alkyl darstellt; wobei (C₁-C₆)Alkyl gegebenenfalls mit (C₁-C₆)Alkyl, OR⁸, Phenyl oder Heteroaryl substituiert sein kann;
R³ H oder (C₁-C₆)Alkyl darstellt; wobei (C₁-C₆)Alkyl gegebenenfalls mit OR⁸ substituiert sein kann;
R⁴ H oder (C₁-C₆) Alkyl darstellt; R⁸ H, (C₁-C₆) Alkyl, Phenyl oder (CH₂)Phenyl darstellt;
wobei Heteroaryl einen 5- bis 7-gliedrigen aromatischen Ring darstellt, der 1 bis 4 Heteroatome enthält, wobei das Heteroatom unabhängig ausgewählt ist aus 0, S und N; wobei das Heteroaryl gegebenenfalls mit einem oder mehr Substituenten, ausgewählt aus (C₁-C₆)Alkyl, Halogen und OR⁸, substituiert sein kann, wobei jeder Substituent gleich oder unterschiedlich sein kann; und
wobei Phenyl gegebenenfalls mit 1 oder mehr Substituenten, ausgewählt aus (C₁-C₆)Alkyl, Halogen und OR⁸, substituiert sein kann, wobei jeder Substituent gleich oder unterschiedlich sein kann;
oder ein pharmazeutisch verträgliches Salz, Solvat oder Polymorph davon, mit der Maßgabe, dass:
Z O ist, wenn A C=O ist.

2. Verbindung der Formel (I) 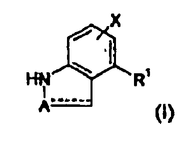 worin A aus N und C=O ausgewählt ist,
X H ist,
----- (die gestrichelte Bindung in Formel (I)) eine Einfachbindung darstellt, wenn A N ist, und nicht vorhanden ist, wenn A C=O ist,
R¹ 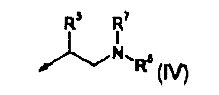 ist,
worin:
R⁵ H, Methyl, Ethyl, Methoxy oder Ethoxy darstellt;
R⁶ (C₁-C₆)Alkyl darstellt;
R⁷ H oder (C₁-C₆)Alkyl darstellt; wobei das (C₁-C₆)Alkyl gegebenenfalls mit 1 oder 2 Substituenten substituiert sein kann, die jeweils unabhängig ausgewählt sind aus OR⁸, Phenyl und substituiertem Phenyl;
R⁸ wie in Anspruch 1 definiert ist;
wobei Phenyl wie in Anspruch 1 definiert ist;
oder ein pharmazeutisch verträgliches Salz, Solvat oder Polymorph davon, mit der Maßgabe, dass
wenn A C=O ist, R⁵ dann nicht H sein kann.

3. Verbindung nach Anspruch 1, worin X ausgewählt ist aus H, OH, F und CN.

4. Verbindung nach Anspruch 1 oder Anspruch 3, worin R¹ Gruppierung (II) ist.

5. Verbindung nach Anspruch 4, worin Z O ist; R² (C₁-C₆)Alkyl ist; R³ ausgewählt ist aus H und (C₁-C₆)Alkyl; und R⁴ H ist.

6. Verbindung nach Anspruch 1 oder Anspruch 3, worin R¹ Gruppierung (III) ist.

7. Verbindung nach Anspruch 6, worin R² (C₁-C₄)Alkyl, gegebenenfalls substituiert mit Phenyl oder Heteroaryl, ist.

8. Verbindung nach Anspruch 2, worin R⁵ aus H und Methoxy ausgewählt ist und R⁷ aus H und Methyl ausgewählt ist.

9. Verbindung, ausgewählt aus:
4-[(2*R*-5*S*)-5-Methyl-4-propylmorpholin-2-yl]-1*H*-indazol;
4-[1-(2-Ethyl)azetidin-3-yl]-1*H*-indazol;
4-[1-(3-Phenylpropyl)azetidin-3-yl]-1*H*-indazol;
4-(1-Propylazetidin-3-yl)-1*H*-indazol;
4-[(2*R*)-4-Propylmorpholin-2-yl]-1,3-dihydro-2*H*-indol-2-on;
4-[(5*S*)-5-Methyl-4-propylmorpholin-2-yl]-1,3-dihydroindol-2-on;
*N*-[2-(1*H*-Indazol-4-yl)ethyl]-*N*-propylamin und
4-[(5*S*)-4-Ethyl-5-methylmorpholin-2-yl]-1*H*-indazol.

10. Verbindung nach einem der Ansprüche 1 bis 9 zur Verwendung als Pharmazeutikum.

11. Verwendung einer Verbindung nach einem der Ansprüche 1 bis 9 bei der Herstellung eines Medikaments für die Behandlung von Sexualdysfunktion.

12. Verwendung nach Anspruch 11, wobei die Sexualdysfunktion männliche erektile Dysfunktion oder weibliche Sexualdysfunktion ist.

13. Verwendung einer Verbindung nach einem der Ansprüche 1 bis 9 bei der Herstellung eines Medikaments für die Behandlung von Depression oder psychiatrischen Störungen.

14. Verwendung einer Verbindung nach einem der Ansprüche 1 bis 9 bei der Herstellung eines Medikaments für die Behandlung von Neurodegeneration.

15. Pharmazeutische Zusammensetzung, umfassend eine Verbindung nach einem der Ansprüche 1 bis 9 und ein pharmazeutisch verträgliches Verdünnungsmittel oder einen pharmazeutisch verträglichen Träger.

## Revendications

1. Composé de formule (I) 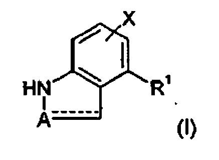 dans laquelle :
A est sélectionné parmi N ou C=O
X est sélectionné parmi H, méthyle, éthyle, OH, OCH₃, OCH₂CH₃, halogène, SCH₃, CN ou CF₃
----- [la liaison en pointillés dans la formule (I)] représente une liaison simple lorsque A représente N, et est absente lorsque A représente C=O
R¹ est sélectionné parmi : 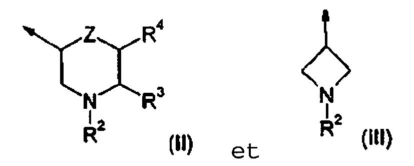
où :
Z représente O ou CH₂ ;
R² représente H ou alkyle en C₁-C₆ ; alkyle en C₁-C₆ pouvant être facultativement substitué par alkyle en C₁-C₆, OR⁸, phényle ou hétéroaryle ;
R³ représente H ou alkyle en C₁-C₆ ; alkyle en C₁-C₆ pouvant être facultativement substitué par OR⁸ ;
R⁴ représente H ou alkyle en C₁-C₆ ;
R⁸ représentant H ou alkyle en C₁-C₆, phényle ou (CH₂)phényle ;
hétéroaryle désignant un noyau aromatique ayant de 5 à 7 éléments et contenant de 1 à 4 hétéroatomes, lesdits hétéroatomes étant indépendamment sélectionnés parmi O, S et N ;
ledit groupe hétéroaryle pouvant être facultativement substitué par 1 ou plusieurs substituants sélectionnés parmi alkyle en C₁-C₆, halogéno et OR⁸, ces substituants pouvant être identiques ou différents ; et
phényle pouvant être facultativement substitué par 1 ou plusieurs substituants sélectionnés parmi alkyle en C₁-C₆, halogéno et OR⁸, ces substituants pouvant être identiques ou différents ;
ou sel, solvate ou polymorphe pharmaceutiquement acceptable correspondant, étant entendu que :
Z représente O lorsque A représente C=O.

2. Composé de formule (I) 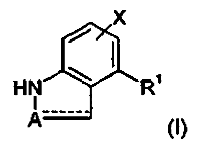 dans laquelle :
A est sélectionné parmi N ou C=O
X représente H
----- [la liaison en pointillés dans la formule (I)] représente une liaison simple lorsque A représente N, et est absente lorsque A représente C=O
R¹ représente 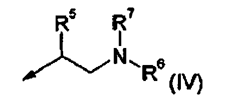 où :
R⁵ représente H, méthyle, éthyle, méthoxy ou éthoxy ;
R⁶ représente alkyle en C₁-C₆ ;
R⁷ représente H ou alkyle en C₁-C₆, ledit groupe alkyle en C₁-C₆ pouvant être facultativement substitué par 1 ou 2 substituants, chacun étant indépendamment sélectionné parmi OR⁸, phényle et phényle substitué ;
R⁸ est tel que défini dans la revendication 1 ;
phényle étant tel que défini dans la revendication 1 ;
ou sel, solvate ou polymorphe pharmaceutiquement acceptable correspondant, étant entendu que :
lorsque A représente C=O, alors R⁵ ne peut pas représenter H.

3. Composé selon la revendication 1, dans lequel X est sélectionné parmi H, OH, F ou CN.

4. Composé selon la revendication 1 ou la revendication 3, dans lequel R¹ est un groupe fonctionnel (II).

5. Composé selon la revendication 4, dans lequel Z représente O ; R² représente alkyle en C₁-C₆ ; R³ est sélectionné parmi H ou alkyle en C₁-C₆ ; et R⁴ représente H.

6. Composé selon la revendication 1 ou la revendication 3, dans lequel R¹ est un groupe fonctionnel (III).

7. Composé selon la revendication 6, dans lequel R² représente alkyle en C₁-C₄ facultativement substitué par phényle ou hétéroaryle.

8. Composé selon la revendication 2, dans lequel R⁵ est sélectionné parmi H et méthoxy ; et R⁷ est sélectionné parmi H et méthyle.

9. Composé sélectionné parmi :
• le 4-[(2*R*,5*S*)-5-méthyl-4-propylmorpholin-2-yl]-1*H*-indazole ;
• le 4-[1-(2-éthyl)azétidin-3-yl]-1H-indazole ;
• le 4-[1-(3-phénylpropyl)azétidin-3-yl]-1H-indazole ;
• le 4-(1-propylazétidin-3-yl)-1H-indazole ;
• la 4-[(2*R*)-4-propylmorpholin-2-yl]-1,3-dihydro-2*H*-indol-2-one ;
• la 4-[(5*S*)-5-méthyl-4-propyl-morpholin-2-yl]-1,3-dihydro-indol-2-one ;
• la *N*-[2-(1*H*-indazol-4-yl)éthyl]-*N*-propylamine ; et
• le 4-[(5*S*)-4-éthyl-5-méthylmorpholin-2-yl]-1*H*-indazole.

10. Composé selon l'une quelconque des revendications 1 à 9, pour une utilisation en tant qu'agent pharmaceutique.

11. Utilisation d'un composé selon l'une quelconque des revendications 1 à 9, dans la préparation d'un médicament pour le traitement de la dysfonction sexuelle.

12. Utilisation selon la revendication 11, dans laquelle la dysfonction sexuelle est la dysfonction érectile masculine ou la dysfonction sexuelle féminine.

13. Utilisation d'un composé selon l'une quelconque des revendications 1 à 9, dans la préparation d'un médicament pour le traitement de la dépression ou de troubles psychiatriques.

14. Utilisation d'un composé selon l'une quelconque des revendications 1 à 9, dans la préparation d'un médicament pour le traitement de la neurodégénérescence.

15. Composition pharmaceutique comprenant un composé selon l'une quelconque des revendications 1 à 9 et un diluant ou support pharmaceutiquement acceptable.
